# EUROPEAN PATENT APPLICATION

(11) **EP 2 216 024 A2**
(43) Date of publication of application: **11.08.2010**
(21) Application number: 10005464.2
(22) Date of filing: 13.02.2008
(51) Int. Cl.: A61K 31/495, A61K 9/00, A61P 9/00, A61P 9/06

(54) **Use of Ranolazine for the treatment of cardiovascular diseases**

(30) Priority: 13.02.2007 US 889734 P; 05.03.2007 US 893121 P; 14.03.2007 US 894903 P; 27.04.2007 US 914645 P; 31.05.2007 US 941219 P; 02.07.2007 US 947613 P
(62) Divisional of application: 08729743.8
(71) Applicant: CV THERAPEUTICS, INC., Palo Alto, CA 94304 (US)
(72) Inventor: Lange, Louis, Palo Alto CA 94304 (US); Jerling, Marcus, 167 56 Bromma (SE); Wolff, Andrew, San Francisco CA 94133 (US)
(74) Representative: Schnappauf, Georg

(57) **Abstract**

Disclosed is the treatment of patients suffering from cardiovascular diseases with an intravenous (IV) infusion of ranolazine. In one embodiment, the IV infusion of ranolazine is followed by an orally administered sustained release ranolazine dosage formulation to maintain human ranolazine plasma levels at therapeutic levels in patients.

## Description

This invention claims priority to U.S. Provisional Patent Application Serial No. 60/889,734, filed February 13, 2007, U.S. Provisional Patent Application Serial No. 60/893,121, filed March 5, 2007. U.S. Provisional Patent Application Serial No. 60/894,903, filed March 14, 2007, U.S. Provisional Patent Application Serial No. 60/914,645, filed April 27, 2007, U.S. Provisional Patent Application Serial No. 60/941,219, filed May 31, 2007, and U.S. Provisional Patent Application Serial No. 60/947,613, filed July 2, 2007, the entireties of each of which is incorporated herein by reference.

### Field of the Invention

This invention relates to methods for treating coronary patients suffering from cardiovascular diseases comprising administering ranolazine to these patients. In one embodiment, the presenting patient suffers from one or more conditions associated with non-ST elevation acute coronary syndrome. In another embodiment, the presenting patient is experiencing an acute coronary event. In an example of this embodiment, this invention provides for a method for titrating the patient to an effective serum ranolazine concentration via an intravenous infusion schedule to achieve therapeutic results. In a further example of this embodiment, this invention provides for long term treatment of a patient with oral ranolazine. In still another embodiment, this invention relates to a method for inhibiting a further non-ST evaluation acute coronary event in a high risk coronary patient previously treated for a non-ST elevation acute coronary event by treating the patient with oral ranolazine. In a further embodiment, this invention provides for treating diabetes by lowering plasma HbA1c in a diabetic, pre-diabetic, or non-diabetic patient suffering from at least one cardiovascular disease comprising administering ranolazine to these patients. In a composition aspect, this invention is directed to an IV formulation suitable for use in the intravenous infusion schedule described above.

### Description of the Art

U.S. Patent No. 4,567,264, the specification of which is incorporated herein by reference in its entirety, discloses ranolazine, (±)-N-(2,6-dimethylphenyl)-4-[2-hydroxy-3-(2-methoxyphenoxy)-propyl]-1-piperazineacetamide, and its pharmaceutically acceptable salts, and their use in the treatment of cardiovascular diseases, including arrhythmias, variant and exercise-induced angina, and myocardial infarction. In its dihydrochloride salt form, ranolazine is represented by the formula:

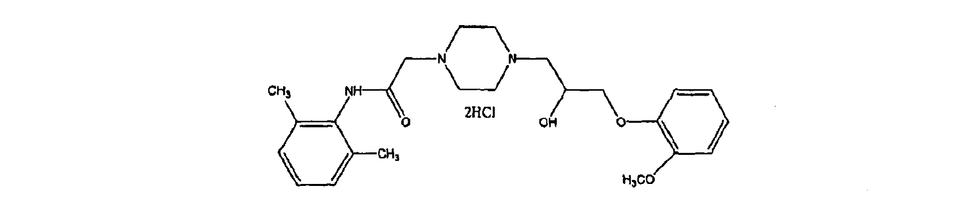

This patent also discloses intravenous (IV) formulations of dihydrochloride ranolazine further comprising propylene glycol, polyethylene glycol 400, Tween 80 and 0.9% saline.

U.S. Patent No. 5,506,229, which is incorporated herein by reference in its entirety, discloses the use of ranolazine and its pharmaceutically acceptable salts and esters for the treatment of tissues experiencing a physical or chemical insult, including cardioplegia, hypoxic or reperfusion injury to cardiac or skeletal muscle or brain tissue, and for use in transplants. Oral and parenteral formulations are disclosed, including controlled release formulations. In particular, Example 7D of U.S. Patent No. 5,506,229 describes a controlled release formulation in capsule form comprising microspheres of ranolazine and microcrystalline cellulose coated with release controlling polymers. This patent also discloses IV ranolazine formulations which at the low end comprise 5 mg ranolazine per milliliter of an IV solution containing about 5% by weight dextrose. And at the high end, there is disclosed an IV solution containing 200 mg ranolazine per milliliter of an IV solution containing about 4% by weight dextrose.

The presently preferred route of administration for ranolazine and its pharmaceutically acceptable salts and esters is oral. A typical oral dosage form is a compressed tablet, a hard gelatin capsule filled with a powder mix or granulate, or a soft gelatin capsule (softgel) filled with a solution or suspension. U.S. Patent No. 5,472,707, the specification of which is incorporated herein by reference in its entirety, discloses a high-dose oral formulation employing supercooled liquid ranolazine as a fill solution for a hard gelatin capsule or softgel.

U.S. Patent No. 6,503,911, the specification of which is incorporated herein by reference in its entirety, discloses sustained release formulations that overcome the problem of affording a satisfactory plasma level of ranolazine while the formulation travels through both an acidic environment in the stomach and a more basic environment through the intestine, and has proven to be very effective in providing the plasma levels that are necessary for the treatment of angina and other cardiovascular diseases.

U.S. Patent No. 6,852,724, the specification of which is incorporated herein by reference in its entirety, discloses methods of treating cardiovascular diseases, including arrhythmias variant and exercise-induced angina and myocardial infarction.

U.S. Patent Application Publication Number 2006/0177502, the specification of which is incorporated herein by reference in its entirety, discloses oral sustained release dosage forms in which the ranolazine is present in 35-50%, preferably 40-45% ranolazine. In one embodiment the ranolazine sustained release formulations of the invention include a pH dependent binder; a pH independent binder; and one or more pharmaceutically acceptable excipients. Suitable pH dependent binders include, but are not limited to, a methacrylic acid copolymer, for example Eudragit^{®} (Eudragit^{®} L100-55, pseudolatex of Eudragit^{®} L100-55, and the like) partially neutralized with a strong base, for example, sodium hydroxide, potassium hydroxide, or ammonium hydroxide, in a quantity sufficient to neutralize the methacrylic acid copolymer to an extent of about 1-20%, for example about 3-6%. Suitable pH independent binders include, but are not limited to, hydroxypropylmethylcellulose (HPMC), for example Methocel^{®} EIOM Premium CR grade HPMC or Methocel^{®} E4M Premium HPMC. Suitable pharmaceutically acceptable excipients include magnesium stearate and microcrystalline cellulose (Avicel^{®} pH101).

In acute or emergency situations in which a patient either is or recently has experienced an acute cardiovascular disease event there is a need to initially and rapidly stabilize the patient. Once the patient has been stabilized there is a need to maintain the patient's stability by providing treatment over an extended period of time.

There is therefore a need for a method for treating patients suffering from an acute cardiovascular disease event comprising administering ranolazine in an intravenous (IV) formulation followed by an oral formulation that provides therapeutically effective plasma concentrations of ranolazine in humans.

### SUMMARY OF THE INVENTION

This invention is directed, in part, to the discovery that rapid infusion of an IV formulation comprising selected concentrations of ranolazine into a patient presenting with one or more conditions associated with non-ST elevation acute coronary syndrome is effective in rapidly treating the condition(s).

In a first aspect, this invention relates to a method for treating a patient suffering from an acute cardiovascular disease event. In a further embodiment of this aspect, the patient suffering from an acute cardiovascular disease event exhibits one or more conditions associated with non-ST elevation acute coronary syndrome. In a further embodiment of this aspect, the patient suffering from an acute cardiovascular disease event exhibits two or more conditions associated with non-ST elevation acute coronary syndrome. In a further embodiment of this aspect, the patient suffering from an acute cardiovascular disease event exhibits three or more conditions associated with non-ST elevation acute coronary syndrome.

In a second aspect, this invention relates to a method for stabilizing a patient suffering from an acute cardiovascular disease event comprising administering an IV solution comprising a selected concentration of ranolazine.

In a third aspect, this invention relates to a method for stabilizing a patient suffering from an acute cardiovascular disease event comprising administering an IV solution of a selected concentration of ranolazine for a period of preferably up to about 96 hours.

In a fourth aspect, this invention relates to a method for treating a stabilized patient suffering from an acute cardiovascular disease event which method comprises administration of an oral sustained release formulation of ranolazine.

In a fifth aspect, this invention relates to a method for treating a patient suffering from an acute cardiovascular disease event, said patient having been stabilized and said patient having to continue to have his/her cardiovascular disease treated after being stabilized.

In a sixth aspect, this invention relates to a method for treating recurrent ischemia in a patient comprising administering an ischemia reducing amount of ranolazine.

In a seventh aspect, this invention relates to a method for treating non-STE myocardial infarction (NSTEMI).

In an eighth aspect, this invention relates to a method for treating unstable angina (UA).

In a ninth aspect, this invention relates to a method for inhibiting a further coronary event associated with acute coronary syndrome in a coronary patient previously treated for a coronary event associated with acute coronary syndrome by treating the patient with oral ranolazine.

In a tenth aspect, this invention relates to the use of an intravenous (IV) infusion (administration) of ranolazine to stabilize a patient suffering from acute cardiovascular conditions followed by oral ranolazine sustained release formulations once the patient is stabilized.

In an eleventh aspect, this invention relates to treating a patient suffering from an acute cardiovascular disease event by a) initiating administration of an IV solution to said patient wherein said IV solution comprises a selected concentration of ranolazine of from about 1.5 to about 3.0 mg per milliliter; b) titrating the IV administration of the IV ranolazine solution to the patient comprising: i) a sufficient amount of the IV solution to provide for about 200 mg of ranolazine delivered to the patient over about a I hour period; ii) followed by either: a sufficient amount of the IV solution to provide for about 80 mg of ranolazine per hour; or if said patient is suffering from renal insufficiency, a sufficient amount of the IV solution to provide for 40 mg of ranolazine per hour; and c) maintaining the titration of b) until the patient has been stabilized which typically occurs within from about 12 to about 96 hours.

In a twelfth aspect, the pH of the IV solution of the eleventh aspect is maintained at a physiologically acceptable pH and the IV solution further comprises either dextrose monohydrate, preferably at a concentration of about 4.6 to about 5.2 weight percent and more preferably at a concentration of about 4.8 to about 5.0 weight percent, or sodium chloride preferably at a concentration of from about 0.8 to about 1.0 weight percent and more preferably at a concentration of about 0.9 weight percent.

In a thirteenth aspect, this invention relates to treating a patient suffering from an acute cardiovascular disease event by a) initiating administration of an IV solution to said patient wherein said IV solution comprises a selected concentration of ranolazine of from about 1.5 to about 3.0 mg per milliliter; b) titrating the IV administration of the IV ranolazine solution to the patient comprising: i) a sufficient amount of the IV solution to provide for about 200 mg of ranolazine delivered to the patient over about a 1 hour period; ii) followed by either: a sufficient amount of the IV solution to provide for about 80 mg of ranolazine per hour; or if said patient is suffering from renal insufficiency, a sufficient amount of the IV solution to provide for about 40 mg of ranolazine per hour; c) maintaining the titration of b) above until the patient has been stabilized which typically occurs within from about 12 to about 96 hours; and d) after completion of the titration in c) above, delivering ranolazine orally to said patient.

In a fourteenth aspect, the pH of the IV solution of the thirteenth aspect is maintained at a physiologically acceptable pH and the IV solution further comprises either dextrose monohydrate, preferably at a concentration of about 4.6 to 5.2 weight percent and more preferably at a concentration of about 4.8 to 5.0 weight percent, or sodium chloride preferably at a concentration of from about 0.8 to 1.0 weight percent and more preferably at a concentration of about 0.9 weight percent.

In a fifteenth aspect, this invention relates to a method for reducing ischemia in a patient prior to coronary intervention. In this method, there is administered to this patient an IV solution which comprises an intravenous formulation of ranolazine, preferably, an ischemia reducing amount, more preferably from about 1.5 to about 3.0 mg of ranolazine per milliliter of IV solution.

In a sixteenth aspect, the pH of the IV solution of the fifteenth aspect is at a physiologically acceptable pH and the IV solution further comprises either dextrose monohydrate, preferably at a concentration of about 4.6 to about 5.2 weight percent and more preferably at a concentration of from about 4.8 to about 5.0 weight percent, or sodium chloride preferably at a concentration of about 0.8 to about 1.0 weight percent and more preferably at a concentration of about 0.9 weight percent.

In a seventeenth aspect, this invention relates to a method for reducing ischemia in a patient undergoing coronary intervention. In this method, there is administered to this patient an IV solution which comprises an ischemia reducing amount of ranolazine, preferably from about 1.5 to about 3.0 mg of ranolazine per milliliter, wherein administration of the IV solution is initiated at least about 4 hours prior and preferably about 6 hours prior to said intervention and further wherein administration of the IV solution is maintained for at least about 4 hours and preferably for at least about 6 hours after said intervention.

In an eighteenth aspect, the pH of the IV solution of the seventeenth aspect is at a physiologically acceptable pH and the IV solution further comprises either dextrose monohydrate, preferably at a concentration of about 4.6 to about 5.2 weight percent and more preferably at a concentration of about 4.8 to about 5.0 weight percent or sodium chloride preferably at a concentration of about 0.8 to about 1.0 weight percent and more preferably at a concentration of about 0.9 weight percent.

In a nineteenth aspect, this invention relates to IV solutions comprising ranolazine concentrations of from about 1.5 to about 3.0 mg ranolazine per milliliter of IV solution. In a further embodiment of this aspect, the pH of this solution is maintained at physiologically acceptable pH and the IV solution further comprises either about 4.6 to about 5.2 weight percent and preferably about 4.8 to about 5.0 weight percent of dextrose monohydrate or about 0.8 to about 1.0 weight percent and preferably about 0.9 weight percent sodium chloride (NaCl) to provide for an isotonic solution.

In a twentieth aspect, this invention provides for a stock aqueous solution of ranolazine which can be added to a standard IV solution container to provide for the requisite concentration of ranolazine. In this aspect, there is provided a 20 cc container comprising a stock ranolazine solution which comprises about 25 mg of ranolazine per milliliter of solution and either about 36 mg of dextrose monohydrate or sufficient sodium chloride to provide for about 0.9 weight percent sodium chloride in the stock solution. In a further embodiment of this aspect, the pH of this stock solution is 4 ± 0.20.

In a twenty-first aspect, this invention provides for one or more drugs which are used in combination with ranolazine.

In a twenty-second aspect, this invention provides for treating patients exhibiting one or more conditions associated with non-ST elevation acute coronary syndrome who also suffer from one or more additional diseases.

In a twenty-third aspect, this invention provides a method of treating bradycardia or bradyarrythmia in a patient comprising administering a bradycardia or bradyarrythmia reducing effective amount of ranolazine. In one instance the bradycardia is a brady cardic episode.

In a twenty-fourth aspect, this invention provides a method of treating ventricular tachycardia or ventricular arrhythmia in a patient comprising administering a ventricular tachycardia or ventricular arrhythmia reducing effective amount of ranolazine.

In a twenty-fifth aspect, this invention provides a method of treating atrial fibrillation in a patient comprising administering an atrial fibrillation reducing effective amount of ranolazine.

A twenty-sixth aspect of this invention is a method of lowering the plasma level of HbA1c in a diabetic, pre-diabetic, or non-diabetic patient suffering from at least one cardiovascular disease, wherein the cardiovascular disease is angina.

A twenty-seventh aspect of this invention is a method of lowering the plasma level of HbA1c in a diabetic, pre-diabetic, or non-diabetic patient suffering from at least one cardiovascular disease, wherein the cardiovascular disease is chronic angina.

A twenty-eighth aspect of this invention is a method of lowering the plasma level of HbA1c in a diabetic, pre-diabetic, or non-diabetic patient suffering from at least one cardiovascular disease, comprising administering a therapeutically effective amount of ranolazine.

A twenty-ninth aspect of this invention is a method of lowering the plasma level of HbA1c in a diabetic, pre-diabetic, or non-diabetic patient suffering from at least one cardiovascular disease, comprising administering from about 250 mg bid to about 2000 mg bid of ranolazine.

A thirtyth aspect of this invention is a method of reducing negative consequences of diabetes comprising administration of ranolazine.

A thirty-first aspect of this invention is a method of delaying or slowing the development of diabetes comprising administration of ranolazine.

A thirty-second aspect of this invention is a method of delaying the initiation of insulin treatment comprising administration of ranolazine.

A thirty-third aspect of this invention is a method of reducing HbA1c levels in a patient without leading to hypoglycemia comprising administration of ranolazine.

A thirty-fourth aspect of this invention is a method of delaying or slowing the development of worsening hyperglycemia in a diabetic, pre-diabetic, or non-diabetic patient suffering from at least one cardiovascular disease, comprising administration of ranolazine.

A thirty-fifth aspect of this invention is a method of reducing or slowing the development of hyperglycemia in a diabetic, pre-diabetic, or non-diabetic patient suffering from at least one cardiovascular disease, comprising administration of ranolazine.

Without being limited to any theory, the selected concentrations of ranolazine in the IV solutions of any aspect of this invention allow the clinician to monitor those patients with renal insufficiency or who develop renal insufficiency so as to quickly titrate the amount of ranolazine downward if the renal insufficiency becomes a clinical issue.

In further aspects this invention relates to:
1. A method for treating a patient suffering from an acute coronary disease event which method comprises administering to said patient an intravenous (IV) solution comprising ranolazine.
2. A method for treating a patient suffering from an acute coronary disease event which event manifests one or more conditions associated with non-ST elevation acute coronary syndrome which method comprises administering to said patient an intravenous solution comprising ranolazine.
3. The method of item 1, wherein the patient suffering from an acute coronary disease event suffers from one or more coronary diseases selected from the group consisting of angina, arrhythmias, intermittent claudication, acute coronary syndrome, heart failure, and recurrent ischemia.
4. The method of item 1, wherein the patient is administered an IV ranolazine solution comprising from about 1.5 to about 3 mg ranolazine per milliliter of solution.
5. The method of item 1, wherein administration of the IV solution to the patient is continued until the patient has been stabilized.
6. The method of item 5, wherein stabilization occurs with from about 12 to about 96 hours after initiation of the IV administration.
7. A method for treating a patient suffering from an acute coronary disease event comprising:
   a) initiating administration of an IV solution of ranolazine to said patient wherein said IV solution comprises a concentration of ranolazine of from about 1.5 to about 3 mg per milliliter,
   b) titrating the IV administration of the IV ranolazine solution to the patient comprising:
      i) a sufficient amount of the IV solution to provide for about 200 mg of ranolazine delivered to the patient over about a 1 hour period;
      ii) followed by either: a sufficient amount of the IV solution to provide for about 80 mg of ranolazine per hour; or if said patient is suffering from renal insufficiency, a sufficient amount of the IV solution to provide for about 40 mg of ranolazine per hour; and
   c) maintaining the titration of b) above until the patient has been stabilized.
8. The method of item 7, wherein the patient is suffering from an acute coronary disease event which event manifests one or more conditions associated with non-ST elevation acute coronary syndrome.
9. The method of item 7, wherein the patient suffering from an acute coronary disease event suffers from one or more diseases selected from the group consisting of angina, arrhythmias, intermittent claudication, acute coronary syndrome, heart failure, and recurrent ischemia.
10. The method of item 7, wherein the patient is administered an IV ranolazine solution comprising from about 1.8 to about 2.2 mg ranolazine per milliliter of solution.
11. The method of item 7, wherein stabilization occurs within from about 12 to about 96 hours after initiation of the IV administration.
12. The method of item 7, wherein the administration of the intravenous formulation of ranolazine is initiated such that a target peak ranolazine plasma concentration of about 2500 ng base/mL is achieved.
13. The method of item 7, wherein after the patient is stabilized, the patient is then transitioned from the intravenous to an oral dose by administering an oral sustained release formulation of ranolazine.
14. The method of item 13, wherein the oral dose of ranolazine is administered about 1 hour prior to the termination of the intravenous infusion of ranolazine.
15. The method of item 13, wherein at the time of transition from intravenous to oral dose, for the intravenous dose of ranolazine of about 80 mg/hr, the oral dose administered is 1000 mg twice daily (2 x 500 mg).
16. The method of item 13, wherein at the time of transition from intravenous to oral dose, for the intravenous dose of ranolazine of about 60 mg/hr, the oral dose administered is 750 mg twice daily (2 x 375 mg).
17. The method of item 13, wherein at the time of transition from intravenous to oral dose, for the intravenous dose of ranolazine of about 40 mg/hr, the oral dose administered is 500 mg twice daily (1 x 500 mg).
18. The method of item 13, wherein at the time of transition from intravenous to oral dose, for the intravenous dose of ranolazine of about 30 mg/hr, the oral dose administered is 375 mg twice daily (1 x 375 mg).
19. The method of item 13, wherein the patient is further administered one or more of the drugs selected from the group consisting of CYP3A inhibitors and P-gp inhibitors.
20. A method for treating a high risk coronary patient presenting with a subsequent acute coronary disease event comprising administring ranolazine to said patient.
21. A method for reducing ischemia associated with coronary intervention in a patient comprising administering an intravenous formulation of ranolazine at least 2 hours prior to intervention.
22. The method of item 21, wherein said coronary intervention is percutaneous coronary intervention.
23. The method of item 22, wherein said percutaneous coronary intervention is selected from the group consisting of percutaneous transluminal coronary angioplasty, implantation of stents, pacemakers, valves, other coronary devices, and coronary artery bypass graft surgery.
24. The method of item 21, wherein the intravenous administration of ranolazine is continued for at least about 2 hours after completion of the intervention.
25. The method of item 21, wherein the patient is administered an IV ranolazine solution comprising from about 1.5 to about 3.0 mg ranolazine per milliliter of solution.
26. A method for treating recurrent ischemia in a patient comprising administering to said patient an ischemia reducing amount of ranolazine.

### DESCRIPTION OF THE FIGURES

Figure 1 shows a graph of the cumulative incidence of death versus days from randomization for patients experiencing no episodes, 1-2 episodes, and >2 episodes of recurrent ischemia.

Figure 2 shows a graph of the incidence of severe recurrent ischemia, myocardial infarction, and cardiovascular death in patients with diabetes or metabolic syndrome presenting with non-ST-Elevation Acute Coronary Syndrome.

Figure 3 shows a graph of the incidence of severe recurrent ischemia, myocardial infarction, and cardiovascular death as a function of TIMI Risk Score and presence of ischemia as detected on Continuous ECG (CECG) monitoring in patients admitted with non-ST-Elevation Acute Coronary Syndrome.

Figure 4 shows the time from randomization to first occurrence of cardiovascular (CV) death, myocardial infarction (MI), or recurrent ischemia for patients on placebo or ranolazine as the number of days to follow-up vs. proportion of patients event-free. The data for this graph is shown below:

| No. at Risk | 0 days | 30 days | 60 days | 90 days | 180 days | 270 days | 360 days | 450 days | 540 days |
|---|---|---|---|---|---|---|---|---|---|
| | | | | | | | | | |
| Placebo | 3281 | 2945 | 2855 | 2791 | 2454 | 1843 | 1223 | 663 | 268 |
| ranolazine | 3279 | 2965 | 2884 | 2814 | 2451 | 1831 | 1223 | 694 | 269 |

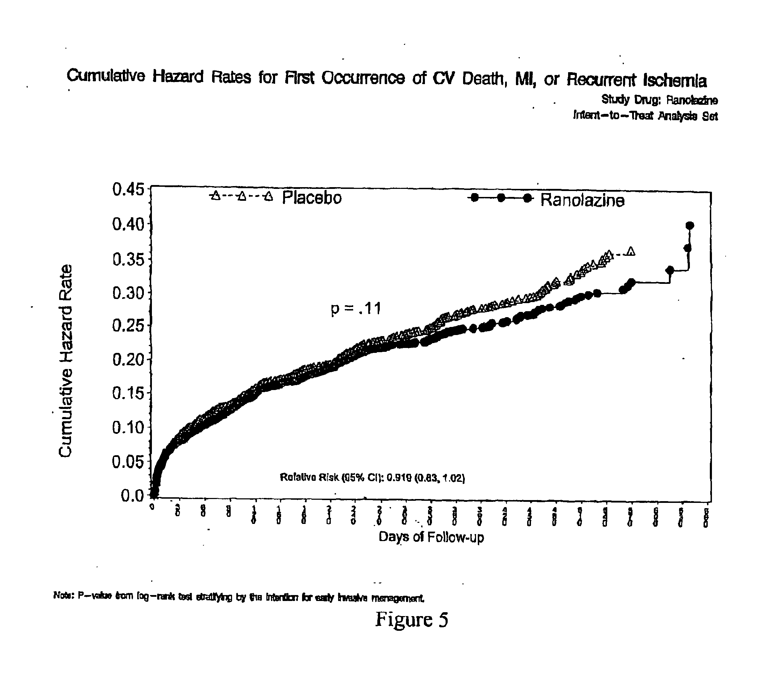

Figure 5 shows the cumulative hazard rates for first occurrence of cardiovascular (CV) death, myocardial infarction (MI), or recurrent ischemia for patients on placebo or ranolazine as the number of days to follow-up vs. cumulative hazard rate. The data for this graph is shown below:

| No. at Risk | 0 days | 30 days | 60 days | 90 days | 180 days | 270 days | 360 days | 450 days | 540 days |
|---|---|---|---|---|---|---|---|---|---|
| | | | | | | | | | |
| Placebo | 3281 | 2945 | 2855 | 2791 | 2454 | 1843 | 1223 | 663 | 268 |
| Ranolazine | 3279 | 2965 | 2884 | 2814 | 2451 | 1831 | 1223 | 694 | 269 |

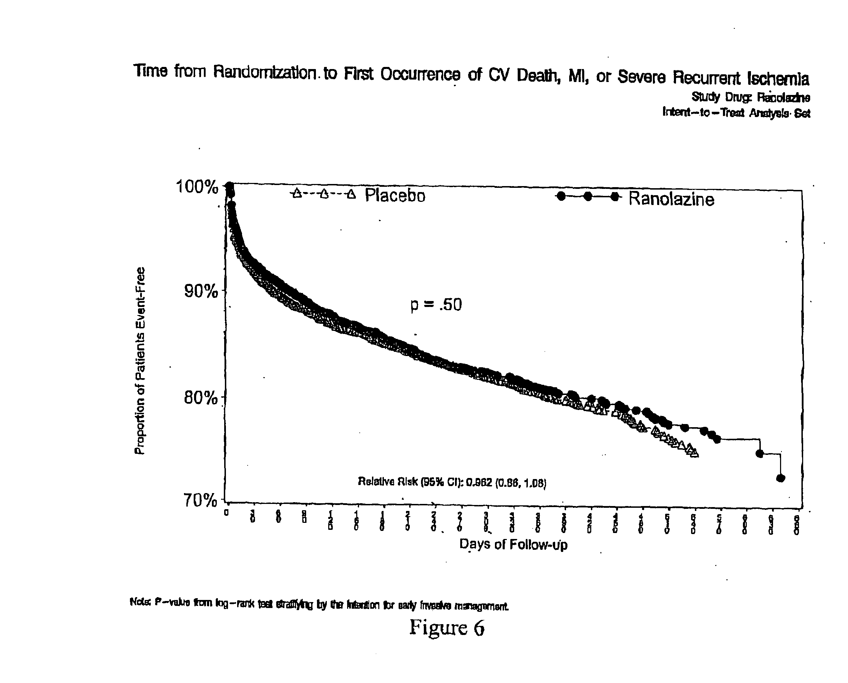

Figure 6 shows the time from randomization to first occurrence of cardiovascular (CV) death, myocardial infarction (MI), or severe recurrent ischemia for patients on placebo or ranolazine as the number of days to follow-up vs. proportion of patients event-free. The data for this graph is shown below:

| No. at Risk | 0 days | 30 days | 60 days | 90 days | 180 days | 270 days | 360 days | 450 days | 540 days |
|---|---|---|---|---|---|---|---|---|---|
| | | | | | | | | | |
| Placebo | 3281 | 2950 | 2869 | 2814 | 2519 | 1925 | 1303 | 716 | 290 |
| Ranolazine | 3279 | 2971 | 2899 | 2838 | 2501 | 1894 | 1278 | 722 | 281 |

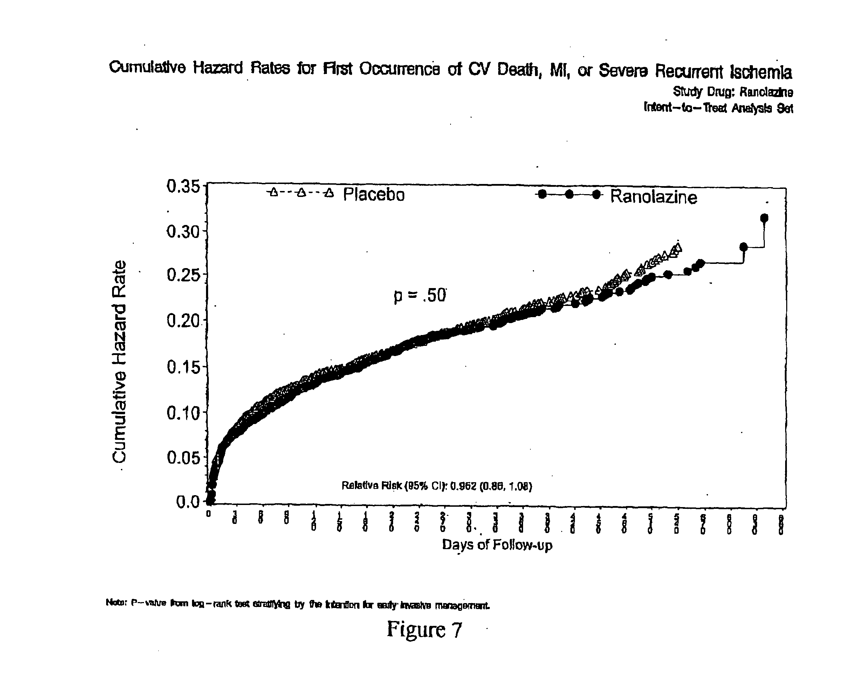

Figure 7 shows the cumulative hazard rates for first occurrence of cardiovascular (CV); death, myocardial infarction (MI), or severe recurrent ischemia for patients on placebo or ranolazine as the number of days of follow-up vs. cumulative hazard rate. The data for this graph is shown below:

| No. at risk | 0 days | 30 days | 60 days | 90 days | 180 days | 270 days | 360 days | 450 days | 540 days |
|---|---|---|---|---|---|---|---|---|---|
| | | | | | | | | | |
| Placebo | 3281 | 2950 | 2869 | 2814 | 2519 | 1925 | 1303 | 716 | 290 |
| Ranolazine | 3279 | 2971 | 2899 | 2838 | 2501 | 1894 | 1278 | 722 | 281 |

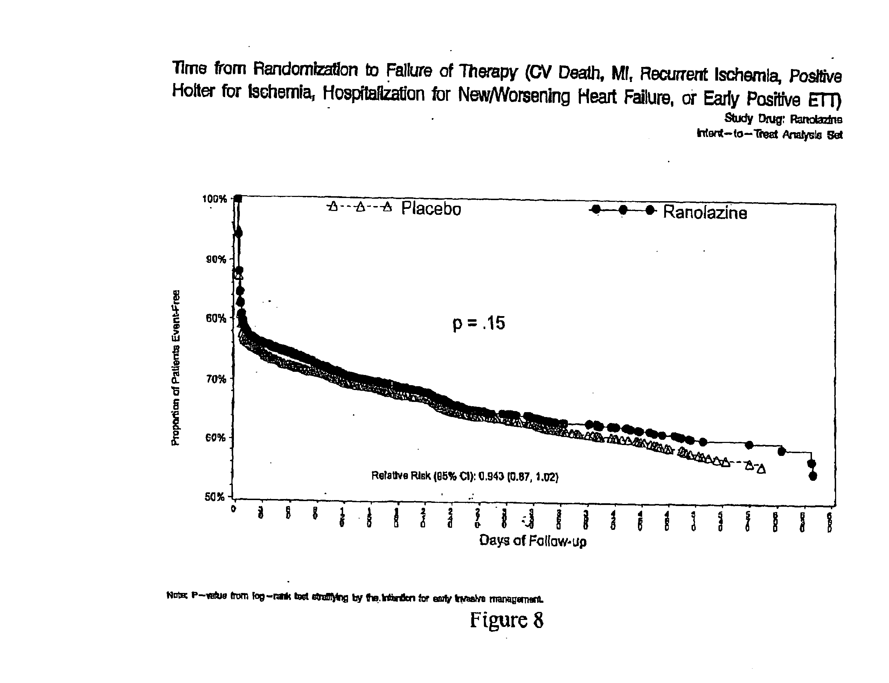

Figure 8 shows the time from randomization to failure of therapy (CV death, MI, recurrent ischemia, positive Holter for ischemia, hospitalization for new/worsening heart failure, or early positive ETT) for patients on placebo or ranolazine as the number of days to follow-up vs. proportion of patients event-free. The data for this graph is shown below:

| No. at risk | 0 days | 30 days | 60 days | 90 days | 180 days | 270 days | 360 days | 450 days | 540 days |
|---|---|---|---|---|---|---|---|---|---|
| | | | | | | | | | |
| Placebo | 3281 | 2391 | 2321 | 2279 | 2009 | 1499 | 992 | 530 | 204 |
| ranolazine | 3279 | 2440 | 2381 | 2317 | 2010 | 1469 | 977 | 546 | 202 |

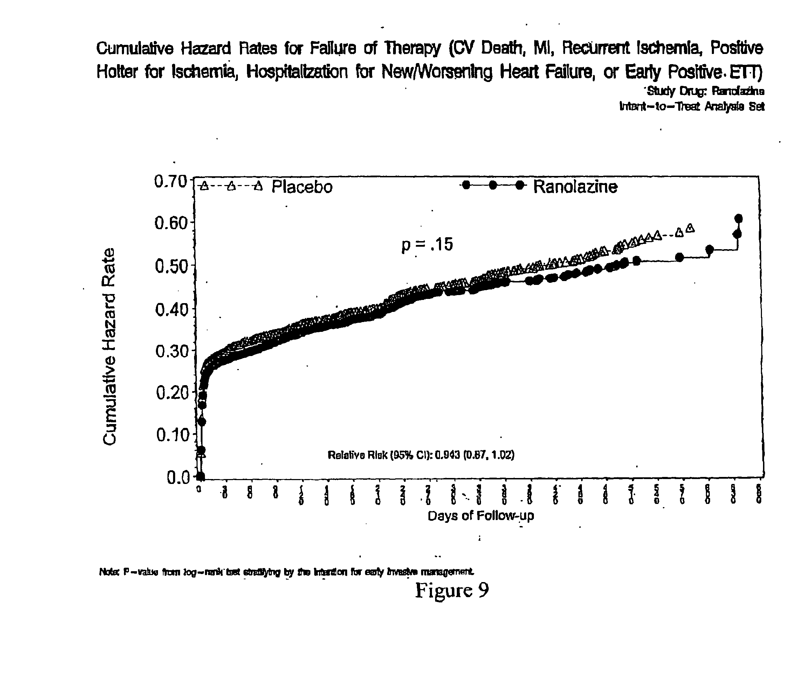

Figure 9 shows the cumulative hazard rates for failure of therapy (CV death, MI, recurrent ischemia, positive Holter for ischemia, hospitalization for new/worsening heart failure, or early positive ETT) for patients on placebo or ranolazine as the number of days of follow-up vs. cumulative hazard rate. The data for this graph is shown below:

| No. at risk | 0 days | 30 days | 60 days | 90 days | 180 days | 270 days | 360 days | 450 days | 540 days |
|---|---|---|---|---|---|---|---|---|---|
| | | | | | | | | | |
| Placebo | 3281 | 2391 | 2321 | 2279 | 2009 | 1499 | 992 | 530 | 204 |
| ranolazine | 3279 | 2440 | 2381 | 2317 | 2010 | 1469 | 977 | 546 | 202 |

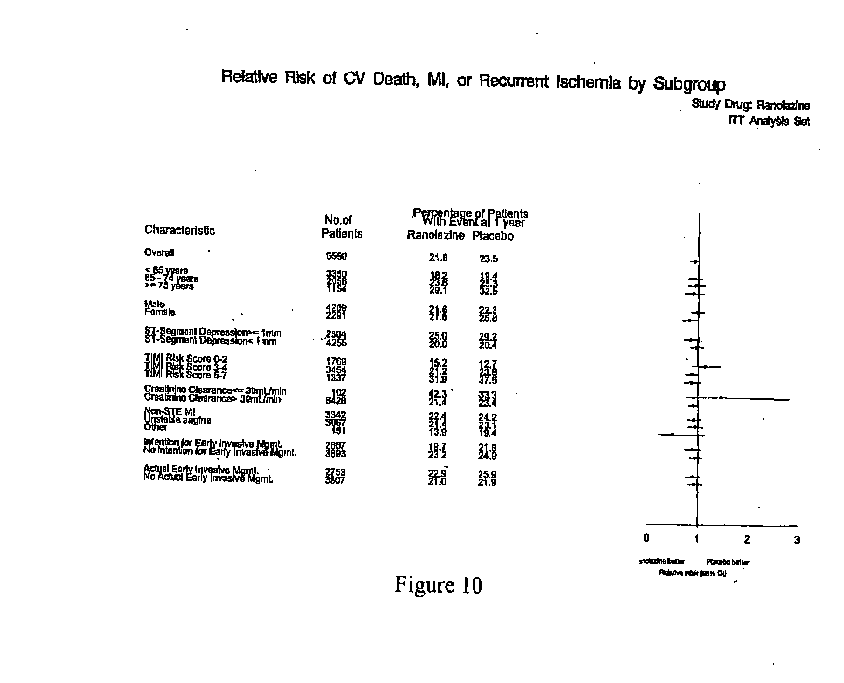

Figure 10 shows the relative risk of CV death, MI, or recurrent ischemia by subgroup as the characteristic, the number of patients with that characteristic and the percentage of patients with event at one year for patients on placebo or ranolazine.

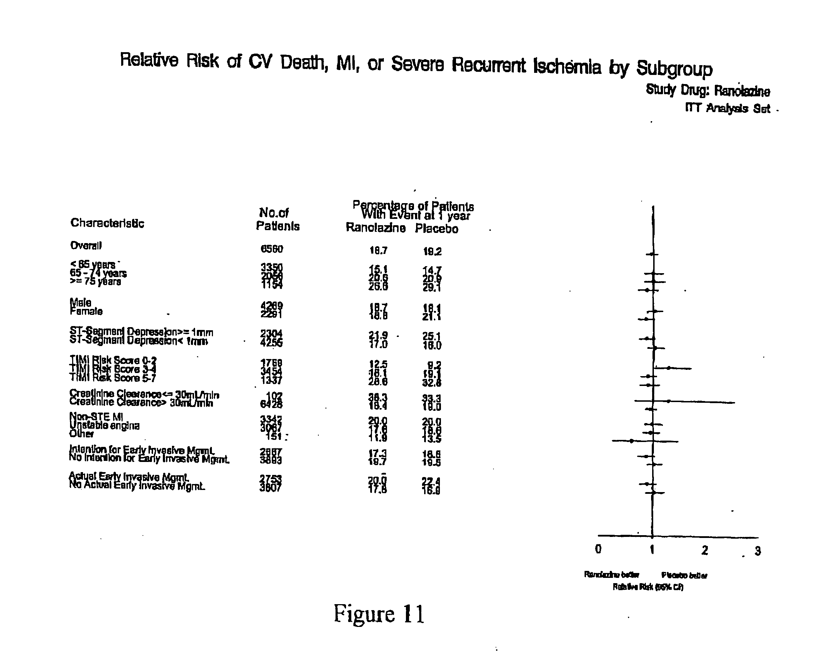

Figure 11 shows the relative risk of CV death, MI, or severe recurrent ischemia by subgroup as the characteristic, the number of patients with that characteristic and the percentage of patients with event at one year for patients on placebo or ranolazine.

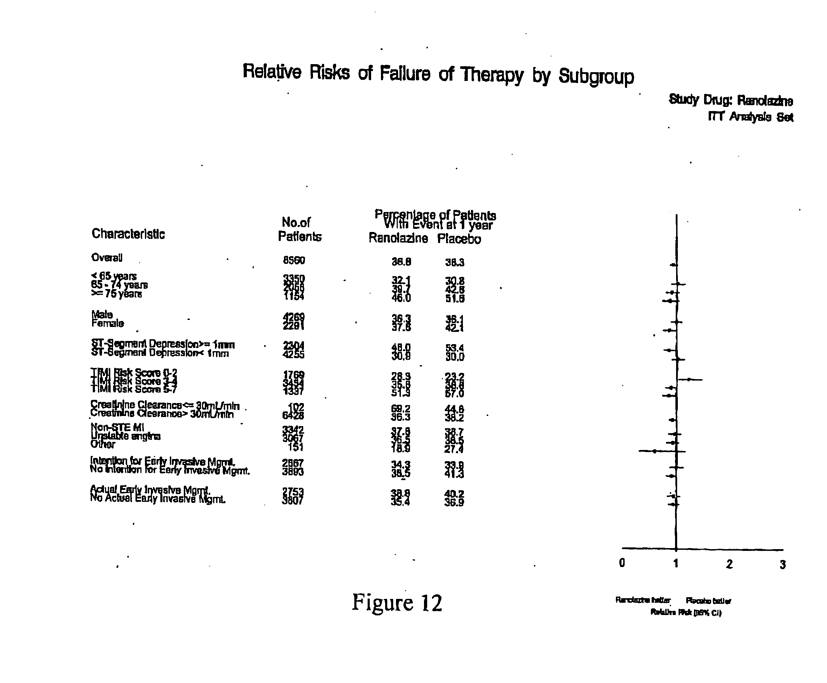

Figure 12 shows the relative risks of failure of therapy by subgroups as the characteristic, the number of patients with that characteristic, and the percentage of patients with event at one year for patients on placebo or ranolazine.

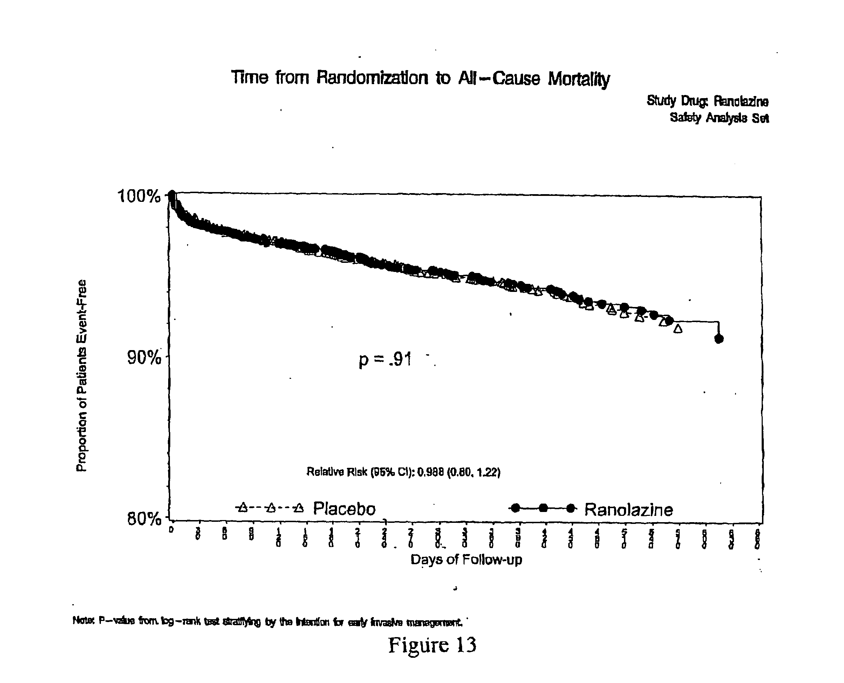

Figure 13 shows the time from randomization to all-cause mortality for patients on placebo or ranolazine as the number of days to follow-up vs. proportion of patients event-free. The data for this graph is shown below:

| No. at risk | 0 days | 30 days | 60 days | 90 days | 180 days | 270 days | 360 days | 450 days | 540 days |
|---|---|---|---|---|---|---|---|---|---|
| | | | | | | | | | |
| Placebo | 3273 | 3157 | 3127 | 3109 | 2851 | 2236 | 1556 | 878 | 367 |
| Ranolazine | 3268 | 3147 | 3121 | 3098 | 2836 | 2218 | 1533 | 889 | 355 |

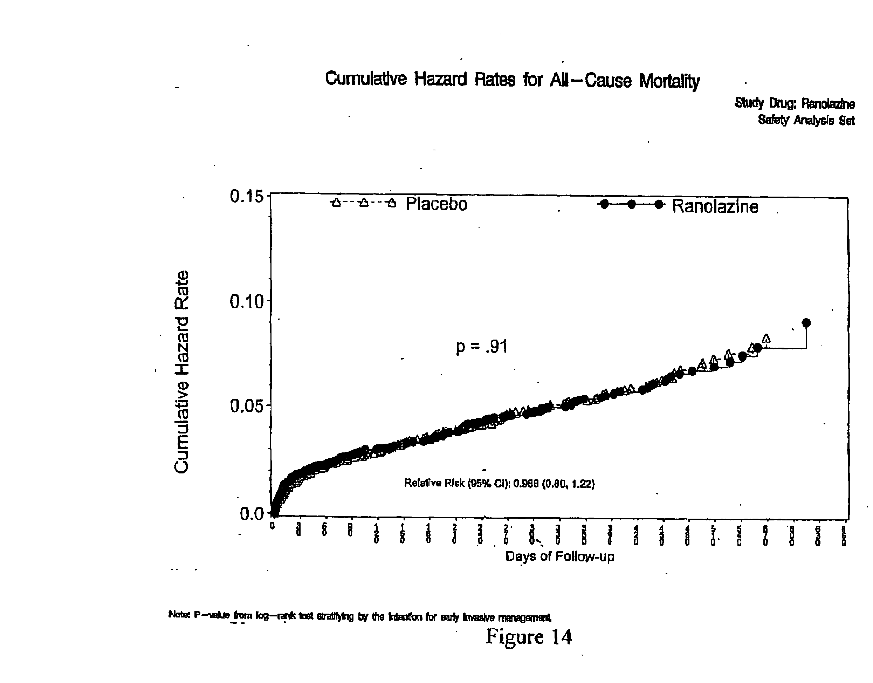

Figure 14 shows the cumulative hazard rates for all-cause mortality for patients on placebo or ranolazine as the number of days of follow-up vs. cumulative hazard rate. The data for this graph is shown below:

| No. at risk | 0 days | 30 days | 60 days | 90 days | 180 days | 270 days | 360 days | 450 days | 540 days |
|---|---|---|---|---|---|---|---|---|---|
| | | | | | | | | | |
| Placebo | 3273 | 3157 | 3127 | 3109 | 2851 | 2236 | 1556 | 878 | 367 |
| Ranolazine | 3268 | 3147 | 3121 | 3098 | 2836 | 2218 | 1533 | 889 | 355 |

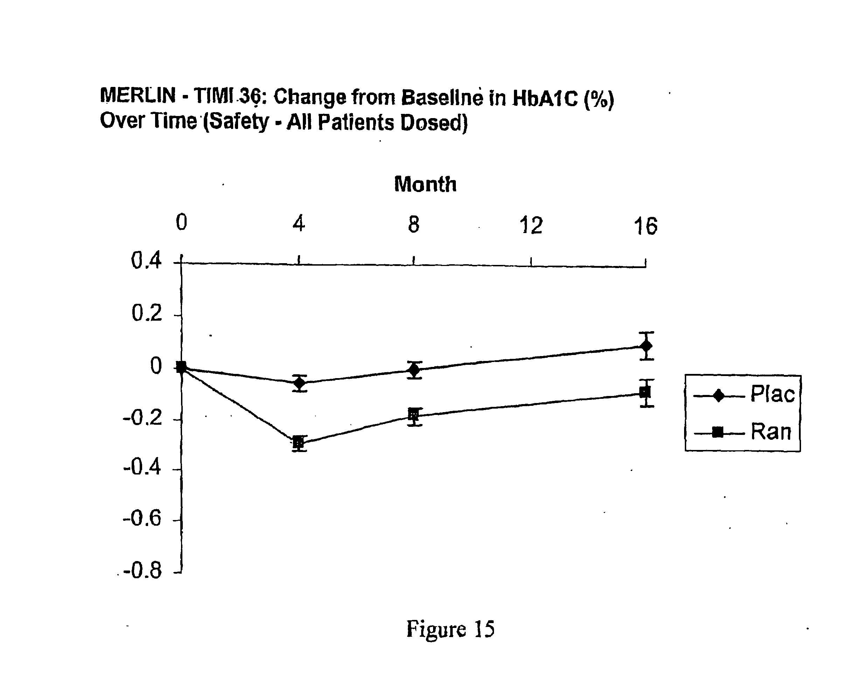

Figure 15 shows the change from baseline in HbA1C (%) over time (safety - all patients dosed) for patients on placebo or ranolazine as the month vs. percentage.

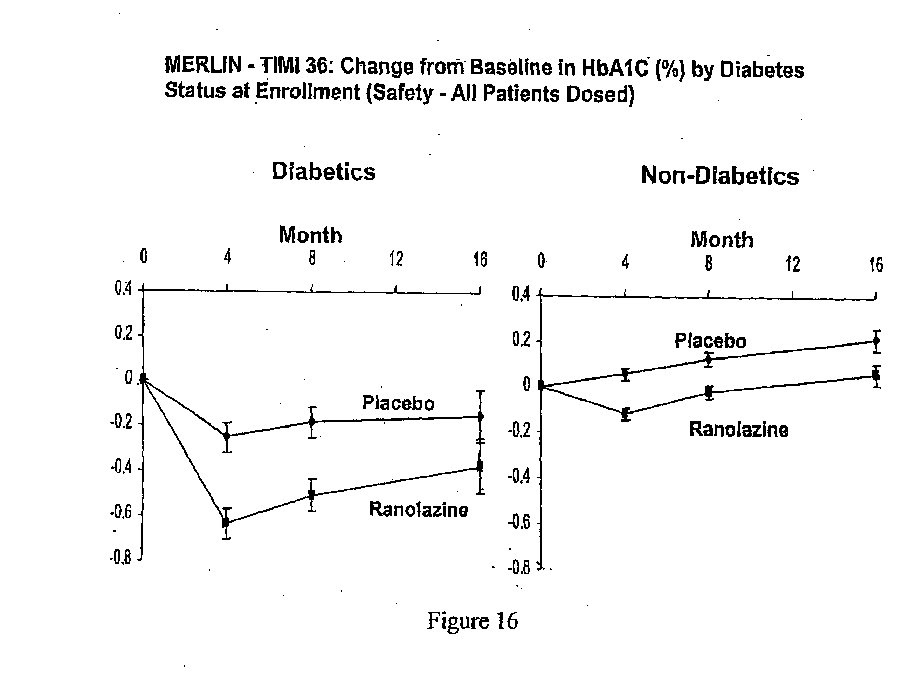

Figure 16 shows the change from baseline in HbA1C (%) by diabetes status at enrollment (safety - all patients doses) for patients on placebo or ranolazine as the month vs. percentage for diabetics or non-diabetics.

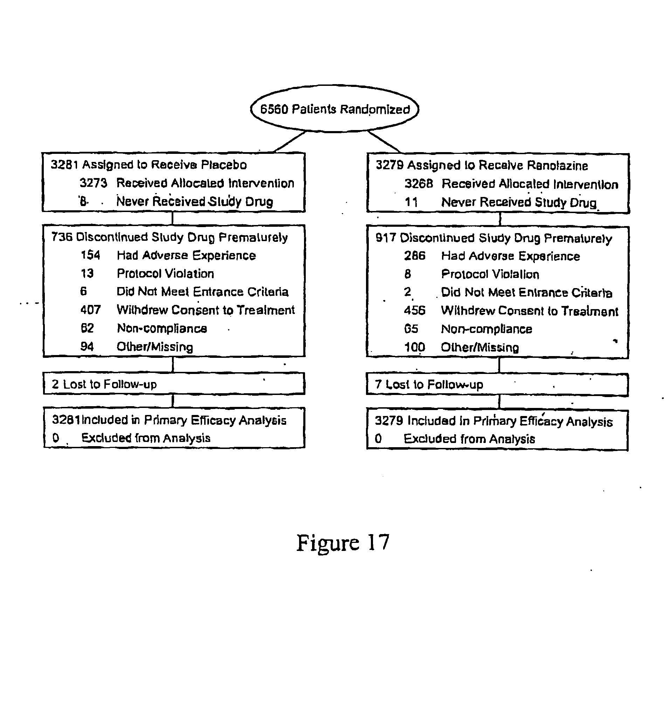

Figure 17 shows the randomization of patients for the MERLIN-TIMI 36 trial.

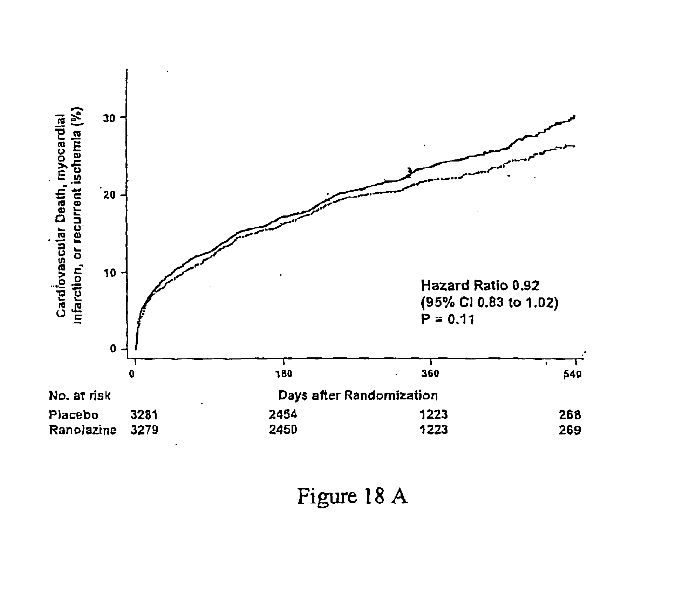

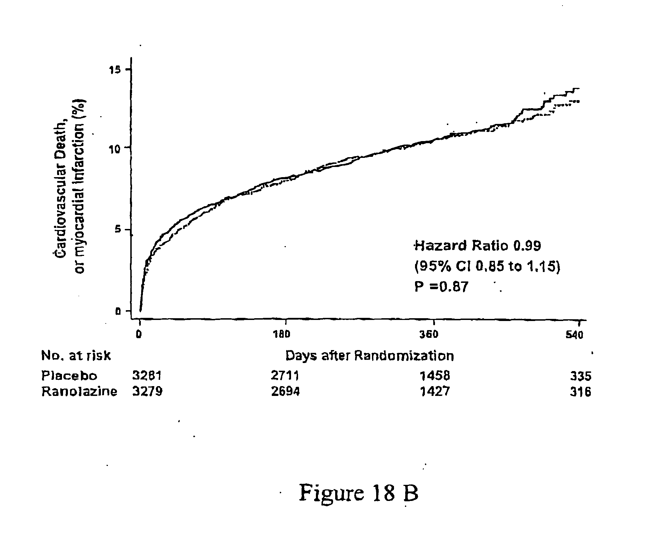

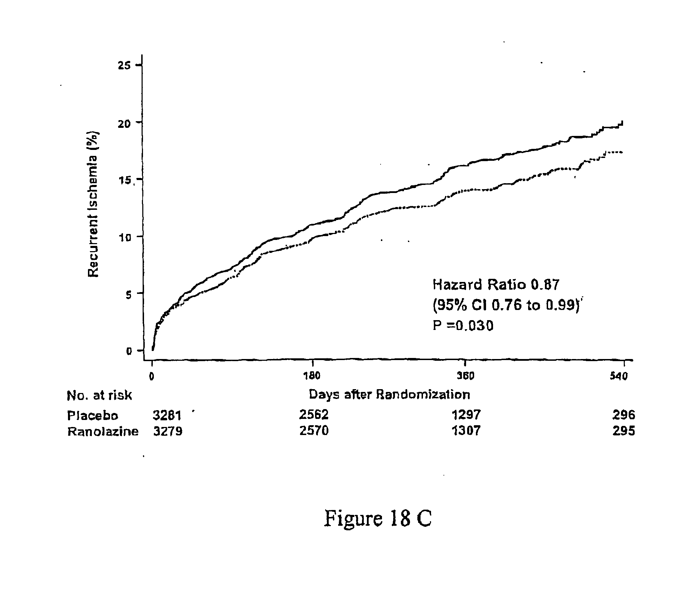

Figure 18 shows the Kaplan-Meier estimated rates of the primary endpoint.
Figure 18A shows endpoint of cardiovascular death, MI, or recurrent ischemia. Figure 18B shows endpoint for cardiovascular death or MI. Figure 18C shows endpoint for recurrent ischemia.

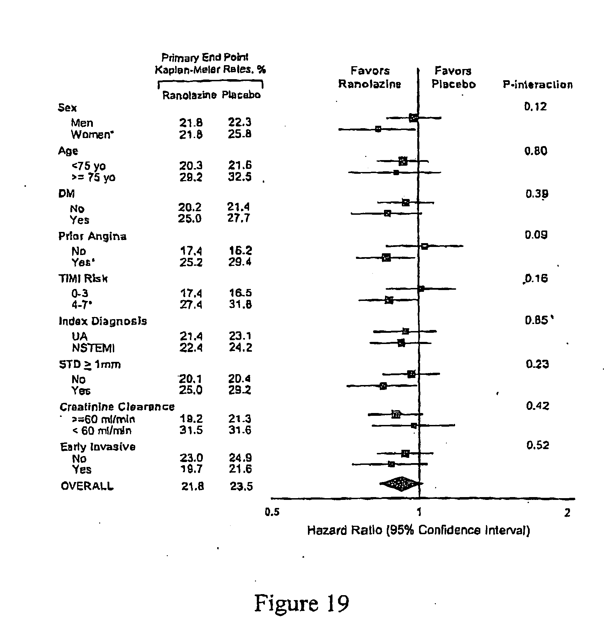

Figure 19 shows the Kaplan-Meier estimated event rates (12 months) and hazard ratios for the primary endpoint in the ranolazine group, as compared with the placebo group in various subgroups. Those subgroups denoted with an asterix were significant at the p <0.0497 level.

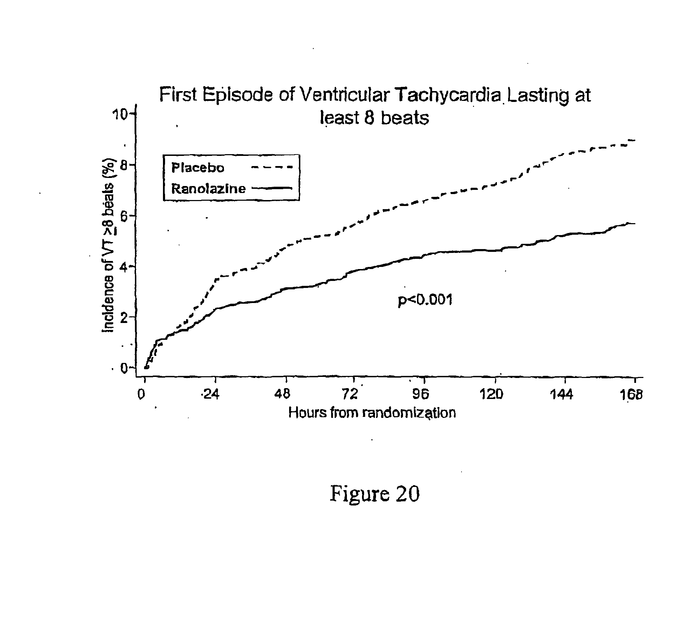

Figure 20 shows the Kaplan-Meier estimated rates of the first occurrence of an episode of ventricular tachycardia lasting at least 8 beats in length.

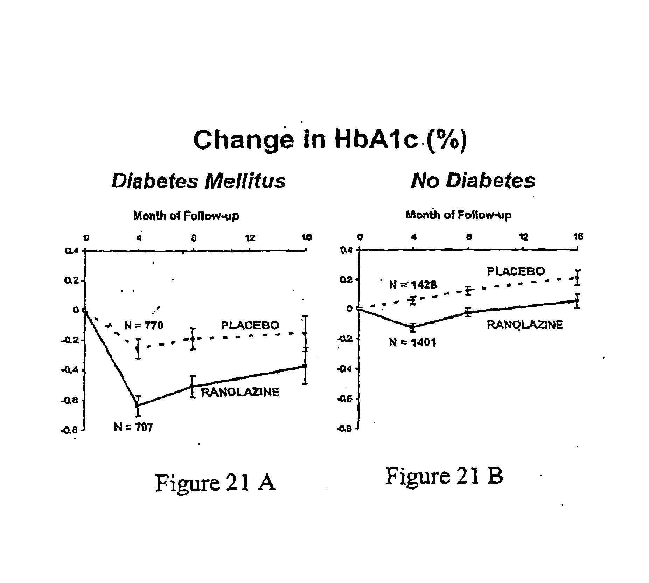

Figure 21 shows the change in HbA1c (%). Figure 21A shows the percentage change in HbA1a in patients diagnosed with diabetes mellitus before or at the start of randomization for this trial versus the months (16) of follow-up. Figure 21A shows

| | M4 | M8 | M16 |
|---|---|---|---|
| Placebo | N=770 | N=598 | N=122 |
| Ranolazine | N=707 | N=535 | N=112 |
| P-value | <0.001 | <0.001 | =0.13 |

Figure 21B shows the percentage change in HbA1c in patients that were either pre-diabetic or non-diabetic at the start of randomization for this trial (had not been diagnosed as diabetic before the start of this trial) versus the months (16) of follow-up.
Figure 21B shows

| | M4 | M8 | M16 |
|---|---|---|---|
| Placebo | N=1428 | N=1113 | N=260 |
| Ranolazine | N=1401 | N=1113 | N=266 |
| P-value | <0.001 | =0.002 | =0.025 |

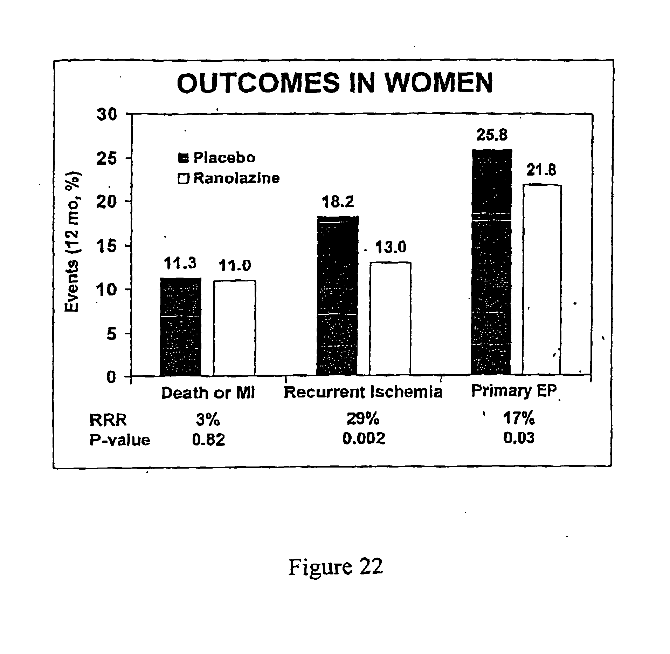

Figure 22 shows the efficacy and safety of ranolazine in women with Non-ST Elevation Acute Coronary Syndromes in MERLIN-TIMI 36. This graph shows the death or MI, recurrent Ischemia, and primary endpoint outcomes events (12 mo., %) in women for placebo and ranolazine.

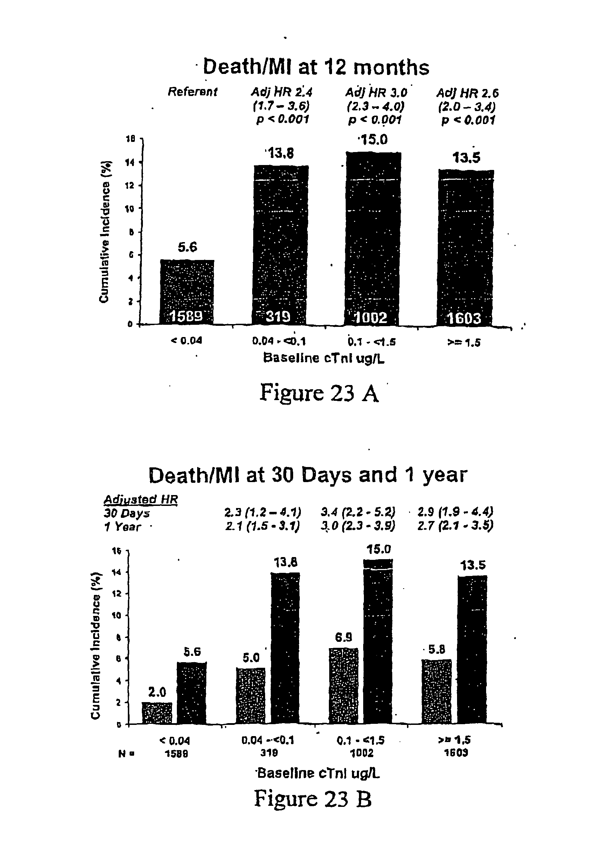

Figure 23A shows the cumulative incidence (%) of death/MI at 12 months vs the baseline cTnI in µg/L. Figure 23B shows the cumulative incidence (%) of death/MI at 30 days and 1 year vs the baseline cTnI in µg/L.

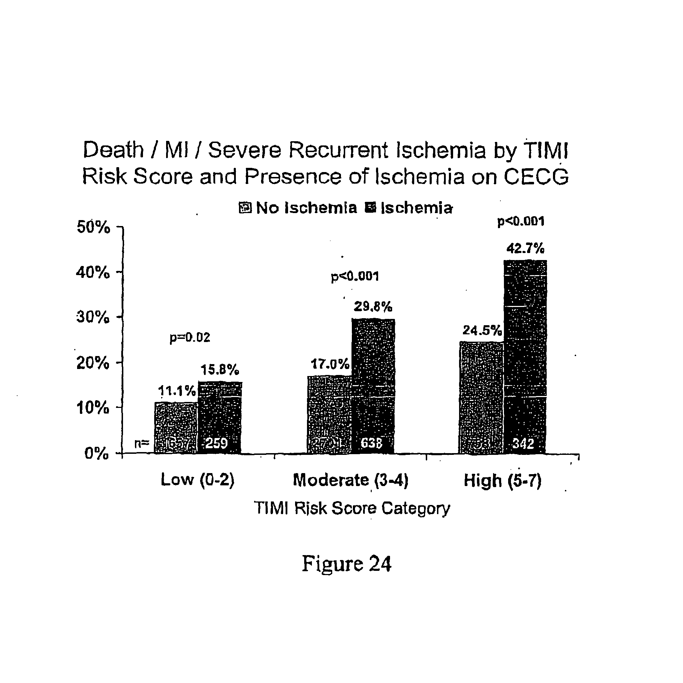

Figure 24 shows the percentage (%) of death/MI/severe recurrent ischemia by TIMI Risk Score and presence of ischemia on CECG.

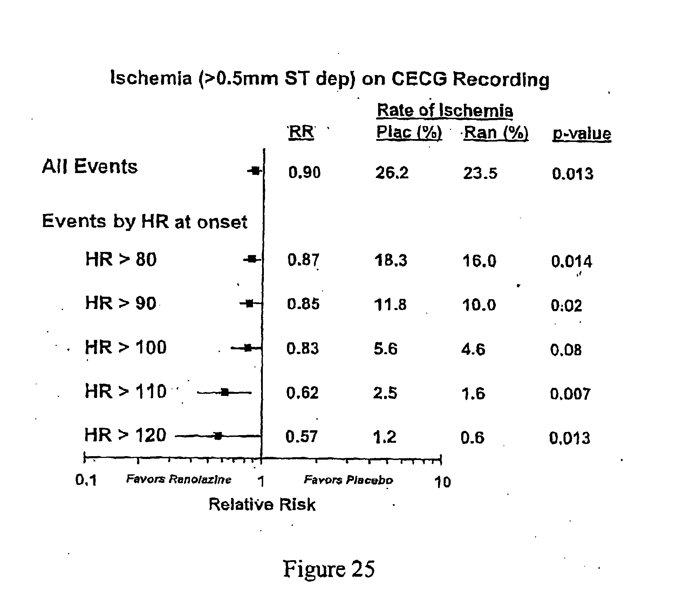

Figure 25 shows the relative risk of ischemia (>0.5 mm ST dep) on CECG recording vs HR.

### DETAILED DESCRIPTION OF THE INVENTION

As noted above, this invention relates to methods for treating coronary patients suffering from cardiovascular diseases comprising administering ranolazine to these patients. However, prior to describing this invention in more detail, the following terms will first be defined.

### Definitions

In this specification and in the claims that follow, reference will be made to a number of terms that shall be defined to have the following meanings.

"Ranolazine" is the compound (±)-N-(2,6-dimethylpbenyl)-4-[2-hydroxy-3-(2-methoxyphenoxy)propyl]-1-piperazine-acetamide, and its pharmaceutically acceptable salts, and mixtures thereof. Unless otherwise stated the ranolazine plasma concentrations used in the specification and examples refer to ranolazine free base. At pH - 4, in an aqueous solution titrated with hydrogen chloride, ranolazine will be present in large part as its dihydrochloride salt.

"Ischemia reducing amount" refers to an amount of ranolazine that decreases oxygen demand without compromising contractile function and affecting heart rate and blood pressure thereby inhibiting ischemia in the treated patient. When coronary intervention is performed, an ischemia reducing amount is preferably an amount of ranolazine, administered as an IV solution, such that about 200 mg of ranolazine is delivered to the patient per hour for at least 4 hours pre- and post-intervention and more preferably about 6 hours pre- and post-intervention.

"Bradycardia or bradyarrythmia reducing effective amount" is an amount of ranolazine that treats the bradycardia or bradyarrythmia.

"Ventricular tachycardia or ventricular arrhythmia reducing effective amount" is an amount of ranolazine that treats ventricular tachycardia or ventricular arrhythmia.

"Atrial fibrillation or atrial fibrillation reducing effective amount" is an amount of ranolazine that treats atrial fibrillation.

"Physiologically acceptable pH" refers to the pH of an intravenous solution which is compatible for delivery into a human patient. Preferably, physiologically acceptable pH's range from about 4 to about 8.5 and preferably from about 4 to 7. Without being limited by any theory, the use of intravenous solutions having a pH of about 4 to 6 are deemed physiologically acceptable as the large volume of blood in the body effectively buffers these intravenous solutions.

"Coronary diseases" or "cardiovascular diseases" refer to diseases of the cardiovasculature arising from any one or more than one of, for example, heart failure, including congestive heart failure, acute heart failure, ischemia, recurrent ischemia, myocardial infarction, arrhythmias (including atrial fibrillation), angina (including exercise-induced angina, variant angina, stable angina, unstable angina), acute coronary syndrome, diabetes, and intermittent claudication. The treatment of such disease states is disclosed in various U.S. patents and patent applications, including U.S. Patent Nos. 6,503,911 and 6,528,511, U.S. Patent Application Serial Nos. 2003/0220344 and 2004/0063717, the complete disclosures of which are hereby incorporated by reference.

"Intermittent claudication" means the pain associated with peripheral artery disease. "Peripheral artery disease" or PAD is a type of occlusive peripheral vascular disease (PVD). PAD affects the arteries outside the heart and brain. The most common symptom of PAD is a painful cramping in the hips, thighs, or calves when walking, climbing stairs, or exercising. The pain is called intermittent claudication. When listing the symptom intermittent claudication, it is intended to include both PAD and PVD.

"An acute coronary disease event" refers to any condition relating to one or more coronary diseases which has/have manifested itself/themselves or has deteriorated to the point where the patient seeks medical intervention typically but not necessarily in an emergency situation.

"Acute coronary syndrome" or "ACS" refers to a range of acute myocardial ischemic states. It encompasses unstable angina and non-ST-segment elevation myocardial infarction (UA/NSTEMI), and ST segment elevation myocardial infarction (STEMI). STEMI refers to a complete occlusion by thrombus. In a preferred embodiment, ACS refers to those patients with a non-ST elevation acute coronary syndrome (NSTEACS). NSTEACS refers to a partial occlusion by the thrombus. NSTEACS is further defined as chest discomfort or anginal equivalent occurring at rest, lasting ≥10 minutes, and consistent with myocardial ischemia, and the presence of ischemic symptoms (≥5 minutes) at rest within 48 hours of admittance which may include index episode, and having at least one of the following indicators of moderate - high risk:
- Elevated cardiac troponin (above local MI limit) or CK-MB (>ULN)
- ST-depression (horizontal or down-sloping) ≥ 0.1 mV
- Diabetes mellitus (requiring insulin or oral therapy)
- A Risk Score of ≥ 3 wherein one point is assigned for each of the following variables and a total score calculated as the arithmetic sum:
   ○ Age ≥ 65 years;
   ○ Known CAD (prior MI, CABG, PCI or angiographic stenosis ≥50%);
   ○ Three or more cardiac risk factors (DM, elevated cholesterol, hypertension, family history);
   ○ More than one episode of ischemic discomfort at rest in the prior 24 hours;
   ○ Chronic aspirin use in the 7 days preceding onset of symptoms;
   ○ ST segment depression ≥ 0.05 mV; and
   ○ Elevated cardiac troponin or CK-MB.

These risk indicators are also referred to as TIMI (thrombolysis in myocardial ischemia) risk factors and are further discussed in Chase, et al., Annals of Emergency Medicine, 48(3):252-259 (2006); Sadanandan, et al., J Am Coll Cardiol., 44(4):799-803 (2004); and Conway, et al., Heart, 92:1333-1334 (2006), each of which is incorporated by reference in its entirety herein.

"Unstable angina" or "UA" refers to a clinical syndrome between stable angina and acute myocardial infarction. This definition encompasses many patients presenting with varying histories and reflects the complex pathophysiological mechanisms operating at different times and with different outcomes. Three main presentations have been described - angina at rest, new onset angina, and increasing angina.

"ECG" refers to an electrocardiogram.

"Cardiovascular intervention" or "coronary intervention" refers to any invasive procedure to treat a coronary disease including, but not limited to, "percutaneous coronary intervention" or PCI. It is contemplated that PCI encompasses a number of procedures used to treat patients with diseases of the heart. Examples of PCI include, but are not limited to, PTCA (percutaneous transluminal coronary angioplasty), implantation of stents, pacemakers, and other coronary devices, CABG (coronary artery bypass graft surgery) and the like.

"Electrical storm" refers to the occurrence of three or more episodes of VT/ventricular fibrillation (VF) within a 24-hour period where each episode is separated by at least 5 minutes. Once commonly used definition of electrical storm is two or more episodes of hemodynamically destabilizing VT/VF occurring in a 24-hour period that usually require electrical cardioversion or defibrillation. Published data suggest that approximately 10-30% of patients with an ICD (implantable cardioverter-defibrillator) experience electrical storm at some point in their clinical course. The majority of patients who experience electrical storm require in-hospital therapy.

"Optional" and "optionally" mean that the subsequently described event or circumstance may or may not occur, and that the description includes instances where the event or circumstance occurs and instances in which it does not. For example, "optional pharmaceutical excipients" indicates that a formulation so described may or may not include pharmaceutical excipients other than those specifically stated to be present, and that the formulation so described includes instances in which the optional excipients are present and instances in which they are not.

"Treating" and "treatment" refer to any treatment of a disease in a patient and include: preventing the disease from occurring in a subject which may be predisposed to the disease but has not yet been diagnosed as having it; inhibiting the disease, i.e., arresting its further development; inhibiting the symptoms of the disease; relieving the disease, i.e., causing regression of the disease, or relieving the symptoms of the disease. In the case of treating arrhythmias, treatment of arrhythmias includes conversion to normal sinus rhythm. The "patient" is a mammal, preferably a human.

"Emergency" refers to an acute situation in which the patient is initially seen by medical personnel. Emergency situations can include, but are not limited to, medical facilities such as hospitals or clinics, emergency rooms at medical facilities such as hospitals or clinics, and emergency situations which involve police and/or medical personnel such as firemen, ambulance attendants, or other medically trained persons.

"Stabilized" refers to a condition in which a patient is not considered to be in immediate risk of morbidity.

"Immediate release" ("IR") refers to formulations or dosage units that rapidly dissolve *in vitro* and are intended to be completely dissolved and absorbed in the stomach or upper gastrointestinal tract. Conventionally, such formulations release at least 90% of the active ingredient within 30 minutes of administration.

"Sustained release" ("SR") refers to formulations or dosage units used herein that are slowly and continuously dissolved and absorbed in the stomach and gastrointestinal tract over a period of about six hours or more. Preferred sustained release formulations are those exhibiting plasma concentrations of ranolazine suitable for no more than twice daily administration with two or less tablets per dosing as described below.

"Intravenous (IV) infusion" or "intravenous administration" refers to solutions or dosage units used herein that are provided to the patient by intravenous route. Such IV infusions can be provided to the patient until for up to about 96 hours in order to stabilize the patient's cardiovascular condition. The method and timing for delivery of an IV infusion is within the skill of the attending medically trained person.

"Renal insufficiency" refers to when a patient's kidneys no longer have enough kidney function to maintain a normal state of health. Renal insufficiency includes both acute and chronic renal failure, including end-stage renal disease (ESRD).

Diabetes, as defined herein, is a disease state characterized by hyperglycemia; altered metabolism of lipids, carbohydrates, and proteins; and an increased risk of complications from vascular disease.

Pre-diabetes, as defined herein, includes people with glucose levels between normal and diabetic have impaired glucose tolerance (IGT). This condition is also called pre-diabetes or insulin resistance syndrome. People with IGT do not have diabetes, but rather have blood glucose levels that are higher than normal but not yet high enough to be diagnosed as diabetes. Their bodies make more and more insulin, but because the tissues don't respond to it, their bodies can't use sugar properly.

Glycemic control is the regulation of blood glucose levels.

Hemoglobin undergoes glycosylation on its amino terminal valine residue to form the glucosyl valine adduct of hemoglobin (HbA1c). The toxic effects of hyperglycemia may be the result of accumulation of such nonenzymatically glycosylated products. The covalent reaction of glucose with hemoglobin also provides a convenient method to determine an integrated index of the glycemic state. For example, the half-life of the modified hemoglobin is equal to that of the erythrocyte (about 120 days). Since the amount of glycosylated protein is proportional to the glucose concentration and the time of exposure of the protein to glucose, the concentration of HbA1c in the circulation reflects the glycemic state over an extended period (4 to 12 weeks) prior to sampling. Thus, a rise in HbA1c from 5% to 10% suggests a prolonged doubling of the mean blood glucose concentration.

### Methods of this invention

As noted previously, in one aspect, this invention provides for a method for treating a patient suffering from an acute cardiovascular disease event. In a further embodiment of this aspect, the patient suffering from acute cardiovascular disease event exhibits one or more conditions associated with non-ST elevation acute coronary syndrome.

Patients presenting themselves with an acute coronary disease event include, but are not limited to, those who are being treated for one or more of the following: angina including stable angina, unstable angina (UA), exercised-induced angina, variant angina, arrhythmias, intermittent claudication, myocardial infarction including non-STE myocardial infarction (NSTEMI), heart failure including congestive (or chronic) heart failure, acute heart failure, or recurrent ischemia.

The methods of this aspect of the invention are preferably achieved by administering to the presenting patient an IV solution comprising a selected concentration of ranolazine. Heretofore, the art provided IV solutions comprising ranolazine which comprised low concentrations of ranolazine (see, e.g., Kluge et al., U.S. Patent No. 4,567,264 where Example 11 of that patent describes using 1.4 mg of ranolazine per mL in an IV solution comprising significant amounts of both propylene glycol (20 g/100 mL) and polyethylene glycol (20 g/100 mL)). Propylene glycol is a viscous liquid as is polyethylene glycol (see, e.g., the Merck Index, 12^{th} Ed., 1996). The increased viscosity resulting from the use of such IV solutions makes the rapid delivery of ranolazine to the patient suffering from an acute cardiovascular disease event more cumbersome and requires that a significant amount of propylene glycol and polyethylene glycol be co-administered.

Alternatively, the art provided IV solutions comprising ranolazine which comprised either high or very high concentrations of ranolazine (either 5 mg/ mL or 200 mg/mL) relative to that employed in the IV solutions used herein. See, e.g., Dow, et al., U.S. Patent No. 5,506,229. In an acute cardiovascular disease event where the patient is suffering from or at risk of suffering from renal insufficiency, the use of such concentrations of ranolazine can result in higher ranolazine plasma levels. Accordingly, the use of such concentrations is contraindicated for treating patients presenting with an acute cardiovascular disease event as the attending physician has little if any time to assess the renal function of that patient prior to initiating treatment.

In the methods of this invention, the IV solution has a selected amount of ranolazine comprising from about 1.5 to 3 mg per milliliter of solution, preferably about 1.8 to 2.2 mg per milliliter and, even more preferably, about 2 mg per milliliter. In contrast to Kluge, et al., *supra*., the IV solution does not contain any propylene glycol or any polyethylene glycol. Rather the compositions of this invention comprise ranolazine, sterile water and dextrose monohydrate or sodium chloride. As such, the compositions of this invention are less viscous than those described by Kluge et al. allowing for more efficient rapid titration of the patient with the IV solution.

The IV solution of this invention is different from the injectable formulations since injectable formulations typically have excipients that may not be needed and may be contraindicated for IV formulations of this invention. For example, an injectable formulation can have an anti-spasmodic agent such as gluconic acid. As such, the IV solutions of this invention do not contain such anti-spasmodic agents and especially gluconic acid.

The IV solution of this invention is used to stabilize a patient suffering from an acute cardiovascular disease event. In particular, the presenting patient is immediately administered this IV solution of ranolazine for a period until the patient is stabilized. Such stabilization typically occurs within from about 12 to about 96 hours.

In a preferred embodiment, the patient suffering from an acute cardiovascular disease event is treated by:
a) initiating administration of an IV solution to said patient wherein said IV solution comprises a selected concentration of ranolazine of from about 1.5 to about 3 mg per milliliter, preferably about 1.8 to about 2.2 mg per milliliter and, even more preferably, about 2 mg per milliliter;
b) titrating the IV administration of the IV ranolazine solution to the patient comprising: i) a sufficient amount of the IV solution to provide for about 200 mg of ranolazine delivered to the patient over about a 1 hour period; ii) followed by either: a sufficient amount of the IV solution to provide for about 80 mg of ranolazine per hour; or if said patient is suffering from renal insufficiency, a sufficient amount of the IV solution to provide for about 40 mg of ranolazine per hour; and
c) maintaining the titration of b) above until the patient stabilizes which typically occurs within from about 12 to about 96 hours.

In one embodiment, the infusion of the intravenous formulation of ranolazine is initiated such that a target peak ranolazine plasma concentration of about 2500 ng base/mL (wherein ng base/mL refers to ng of the free base of ranolazine/mL) is achieved.

The downward adjustment of ranolazine infusion for a patient experiencing adverse events deemed to be treatment related, is within the knowledge of the skilled in the art and, based on the concentration ofranolazine in the IV solution, easy to achieve. Adverse events in addition to those described above include, but are not limited to, profound and persistent QTc prolongation, not attributed to other reversible factors such as hypokalemia; dizziness; nausea/vomiting; diplopia; parasthesia; confusion; and orthostatic hypotension. In one embodiment, the dose of intravenous solution of ranolazine may be adjusted to a lower dose such as, but not limited to, about 60 mg/hr, about 40 mg/hr, or about 30 mg/hr. In another embodiment, the intravenous delivery of ranolazine may be temporarily discontinued for 1-3 hrs and then restarted at the same or lower dose for patients experiencing adverse events deemed to be treatment related.

In a preferred embodiment, once stabilized the patient is then administered an oral sustained release formulation of ranolazine. Specifically, this invention is particularly useful for treating a high risk coronary disease patient with a subsequent acute coronary disease event by treating a patient with ranolazine. A high risk coronary patient is one who previously had at least one acute coronary disease event. In a preferred embodiment, a high risk patient has a TIMI risk score of 3 or higher.

In one embodiment, the oral dose of ranolazine is administered about 1 hour prior to the termination of the intravenous infusion of ranolazine. In one aspect of this embodiment, at the time of transition from intravenous to oral dose, for the intravenous dose of ranolazine of about 80 mg/hr, the oral dose administered is 1000 mg once or twice daily (2 x 500 mg). In another aspect of this embodiment, at the time of transition from intravenous to oral dose, for the intravenous dose of ranolazine of about 60 mg/hr, the oral dose administered is 750 mg once or twice daily (2 x 375 mg). In still another aspect of this embodiment, at the time of transition from intravenous to oral dose, for the intravenous dose of ranolazine of about 40 mg/hr, the oral dose administered is 500 mg (1 x 500 mg). In still another aspect of this embodiment, at the time of transition from intravenous to oral dose, for the intravenous dose of ranolazine of about 30 mg/hr, the oral dose administered is 375 mg (1 x 375 mg).

The downward adjustment of the oral dose for a patient experiencing adverse events deemed to be treatment related, is also within the knowledge of the skilled in the art. For example, the oral dose of ranolazine can be adjusted for patients with newly developed severe renal insufficiency. Other adverse events include, but are not limited to, profound and persistent QTc prolongation, not attributed to other reversible factors such as hypokalemia; dizziness; nausea/vomiting; diplopia; parasthesia; confusion; and orthostatic hypotension. In one embodiment, the oral dose of ranolazine may be adjusted downward to 500 mg once or twice daily, if not already at this dose or lower. In one embodiment, the oral dose of ranolazine may be adjusted to the next lower dose such as, but not limited to, 750 mg once or twice daily, 500 mg once or twice daily, or 375 mg once or twice daily.

In one embodiment, a starting oral dose of 375 mg once or twice daily may be administered to a patient treated with moderate CYP3A inhibitors, such as, diltiazem >180 mg/day, fluconazole and the like, and P-gp inhibitors such as, verapamil, cyclosporine and the like. In one embodiment, the 1000 mg oral dose of ranolazine is administered such that a mean peak ranolazine plasma concentration of about 2500 ng base/mL ± 1000 ng base/mL is achieved.

In one embodiment, the invention relates to a method for reducing ischemia associated with cardiovascular intervention in a patient comprising intravenously administering an intravenous formulation of ranolazine from at least about 4 hours to about 12 hours prior to intervention and preferably about 6 hours prior to intervention. In a further aspect of this embodiment, the invention further comprises continuing to administer the ranolazine intravenously for a period of from about 2 hours to about 12 hours after intervention, preferably for at least about 4 hours and more preferably about 6 hours after completion of the intervention.

In a preferred embodiment, a patient receives intravenous ranolazine for at least about 4 hours or at least about 6 hours prior to the intervention and then receives intravenous ranolazine for at least about 4 hours or at least about 6 hours after intervention.

In these embodiments of the invention, the ranolazine intravenously administered is a intravenous formulation as described herein.

It is also contemplated that the methods of this invention will also reduce other types of ischemia, such as cerebral ischemia, renal ischemia, ischemia associated with organ transplant and the like. In those embodiments, the evaluation and or therapy may include, but is not limited to, treatment of arteriovenous malformations, repair of aneurysms, including abdominal aortic aneurysms and cerebral aneurysms, repair of endoleaks after aneurysm treatment, and the like.

It is contemplated that by administering ranolazine prior to the therapy, the ischemia associated therewith is reduced. To measure ischemia, a patient is fitted with a Holter monitor.

Without limiting the scope of the invention, the formulations of the invention can be used for treating various diseases, such as, cardiovascular diseases e.g., arteriosclerosis, hypertension, arrhythmia (e.g. ischemic arrhythmia, arrhythmia due to myocardial infarction, myocardial stunning, myocardial dysfunction, arrhythmia after PTCA or after thrombolysis, etc.), angina pectoris, cardiac hypertrophy, myocardial infarction, heart failure (e.g., congestive heart failure, acute heart failure, cardiac hypertrophy, etc.), restenosis after PTCA, PTCI (percutaneous transluminal coronary intervention), electrical storm, and shock (e.g., hemorrhagic shock, endotoxin shock, etc.); renal diseases e.g., diabetes mellitus, diabetic nephropathy, ischemic acute renal insufficiency, etc.; organ disorders associated with ischemia or ischemic reperfusion e.g., heart muscle ischemic reperfusion associated disorders, acute renal insufficiency, or disorders induced by surgical treatment such as CABG (coronary artery bypass grafting) surgeries, vascular surgeries, organ transplantation, non-cardiac surgeries or PTCA; cerebrovascular diseases e.g., ischemic stroke, hemorrhagic stroke, etc.; cerebro ischemic disorders e.g., disorders associated with cerebral infarction, disorders caused after cerebral apoplexy such as sequelae, or cerebral edema; and ischemia induced in donor tissues used in transplants where donor tissues include but are not limited to, renal transplants, skin grafts, cardiac transplants, lung transplants, corneal transplants, and liver transplants. The formulations of this invention can also be used as an agent for myocardial protection during CABG surgeries, vascular surgeries, PTCA, PTCI, organ transplantation, or non-cardiac surgeries.

Preferably, the formulations of this invention can be used for myocardial protection before, during, or after CABG surgeries, vascular surgeries, PTCA, organ transplantation, or non-cardiac surgeries. Preferably, the formulations of this invention can be used for myocardial protection in patients presenting with ongoing cardiac (acute coronary syndromes, e.g., myocardial infarction or unstable angina) or cerebral ischemic events (e.g., stroke). Preferably, the formulations of this invention can be used for chronic myocardial protection in patients with diagnosed coronary heart disease (e.g., previous myocardial infarction or unstable angina) or patients who are at high risk for myocardial infarction (age greater than 65 and two or more risk factors for coronary heart disease).

### Compositions of the invention

### Intravenous Formulation

In one aspect, the invention provides an intravenous (IV) solution comprising a selected concentration of ranolazine. Specifically, the IV solution preferably comprises about 1.5 to about 3.0 mg of ranolazine per milliliter of a pharmaceutically acceptable aqueous solution, more preferably about 1.8 to about 2.2 mg and even more preferably about 2 mg. In order to allow for the rapid intravenous flow of ranolazine into the patient, the IV solution preferably contains no viscous components including by way of example as propylene glycol or polyethylene glycol (e.g., polyethylene glycol 400). It is understood that minor amounts of viscous components that do not materially alter the viscosity may be included in the intravenous formulations of this invention. In a particularly preferred embodiment, the viscosity of the IV solution is preferably less than 10 cSt (centistokes) at 20°C, more preferably less than 5 cSt at 20°C and even more preferably less than 2 cSt at 20°C.

In one embodiment, the IV solution comprises:
about 1.5 to about 3.0 mg of ranolazine per mL of IV solution; and
either about 4.8 to about 5.0 weight percent dextrose or about 0.8 to about 1.0 weight percent sodium chloride.

In one embodiment, the IV solution comprises:
about 1.8 to about 2.2 mg of ranolazine per mL of IV solution; and
either about 4.8 to about 5.0 weight percent dextrose or about 0.8 to about 1.0 weight percent sodium chloride.

In one embodiment, the IV solution of this invention comprises:
about 2 mg of ranolazine per mL of IV solution; and
either about 4.8 to about 5.0 weight percent dextrose or about 0.9 weight percent sodium chloride.

The IV solutions described herein can be prepared from a stock solution comprising a 20 mL container for single use delivery which container comprises a sterile aqueous solution of ranolazine at a concentration of about 25 mg/mL; either about 36 mg/mL dextrose monohydrate or about 0.9 weight percent sodium chloride; and having a pH of about 4. Surprisingly, employing such high concentrations of ranolazine and dextrose monohydrate or ranolazine and sodium chloride in the stock solutions provide for compositions which are stable and have adequate shelf-lives, preferably of greater than 6 months.

Exemplary methods for preparing the stock solutions are described in Examples 2 and 3.

In a typical setting, two 20 mL containers described herein are injected into an IV container containing 460 mL of sterile saline (0.9 weight percent (w%) sodium chloride) or an aqueous dextrose solution (water containing 5 weight percent dextrose monohydrate) to provide for an IV solution of about 2 mg/mL of ranolazine maintained at physiologically acceptable pH. Containers useful herein include, but are not limited to, vials, syringes, bottles, IV bags, and the like.

In another embodiment, the intravenous formulation as above, is diluted with a sterile diluent prior to use. In one embodiment, the sterile diluent is 5 % dextrose or a 0.9 weight percent saline solution. In one embodiment, the intravenous formulation is further diluted into bags of sterile diluent.

### Oral Formulation

In one embodiment, a formulation of ranolazine is an oral formulation. In one embodiment, an oral formulation of ranolazine is a tablet. In one embodiment, the tablet of ranolazine is up to 500 mg. In a preferred embodiment, the ranolazine tablet is 375 mg, and/or 500 mg.

The oral formulation of ranolazine is thoroughly discussed in U.S. Patent No. 6,303,607 and U.S. Publication No. 2003/0220344, which are both incorporated herein by reference in their entirety.

The oral sustained release ranolazine dosage formulations of this invention are administered one, twice, or three times in a 24 hour period in order to maintain a plasma ranolazine level above the threshold therapeutic level and below the maximally tolerated levels, which is preferably a plasma level of about 550 to 7500 ng base/mL in a patient.

In a preferred embodiment, the plasma level of ranolazine ranges about 1500-3500 ng base/mL.

In order to achieve the preferred plasma ranolazine level, it is preferred that the oral ranolazine dosage forms described herein are administered once or twice daily. If the dosage forms are administered twice daily, then it is preferred that the oral ranolazine dosage forms are administered at about twelve hour intervals.

In addition to formulating and administering oral sustained release dosage forms of this invention in a manner that controls the plasma ranolazine levels, it is also important to minimize the difference between peak and trough plasma ranolazine levels. The peak plasma ranolazine levels are typically achieved at from about 30 minutes to eight hours or more after initially ingesting the dosage form while trough plasma ranolazine levels are achieved at about the time of ingestion of the next scheduled dosage form. It is preferred that the sustained release dosage forms of this invention are administered in a manner that allows for a peak ranolazine level no more than 8 times greater than the trough ranolazine level, preferably no more than 4 times greater than the trough ranolazine level, preferably no more than 3 times greater than the trough ranolazine level, and most preferably no greater than 2 times trough ranolazine level.

The sustained release ranolazine formulations of this invention provide the therapeutic advantage of minimizing variations in ranolazine plasma concentration while permitting, at most, twice-daily administration. The formulation may be administered alone, or (at least initially) in combination with an immediate release formulation if rapid achievement of a therapeutically effective plasma concentration of ranolazine is desired or by soluble IV formulations and oral dosage forms.

### Combination Therapy

Coronary patients being treated for an acute cardiovascular disease event by administration of ranolazine often exhibit diseases or conditions that benefit from treatment with other therapeutic agents. These diseases or conditions can be of the cardiovascular nature or can be related to pulmonary disorders, metabolic disorders, gastrointestinal disorders and the like. Additionally, some coronary patients being treated for an acute cardiovascular disease event by administration of ranolazine exhibit conditions that can benefit from treatment with therapeutic agents that are antibiotics, analgesics, and/or antidepressants and anti-anxiety agents.

### Cardiovascular Agent Combination Therapy

Cardiovascular related diseases or conditions that can benefit from a combination treatment of ranolazine with other therapeutic agents include, without limitation, angina including stable angina, unstable angina (UA), exercised-induced angina, variant angina, arrhythmias, intermittent claudication, myocardial infarction including non-STE myocardial infarction (NSTEMI), heart failure including congestive (or chronic) heart failure, acute heart failure, or recurrent ischemia.

Therapeutic agents suitable for treating cardiovascular related diseases or conditions include anti-anginals, heart failure agents, antithrombotic agents, antiarrhythmic agents, antihypertensive agents, and lipid lowering agents.

The co-administration of ranolazine with therapeutic agents suitable for treating cardiovascular related conditions allows enhancement in the standard of care therapy the patient is currently receiving.

### Anti-anginals

Anti-anginals include beta-blockers, calcium channel blockers, and nitrates. Beta blockers reduce the heart's need for oxygen by reducing its workload resulting in a decreased heart rate and less vigorous heart contraction. Examples of beta-blockers include acebutolol (Sectral), atenolol (Tenormin), betaxolol (Kerlone), bisoprolol/hydrochlorothiazide (Ziac), bisoprolol (Zebeta), carteolol (Cartrol), esmolol (Brevibloc), labetalol (Normodyne, Trandate), metoprolol (Lopressor, Toprol XL), nadolol (Corgard), propranolol (Inderal), sotalol (Betapace), and timolol (Blocadren).

Nitrates dilate the arteries and veins thereby increasing coronary blood flow and decreasing blood pressure. Examples of nitrates include nitroglycerin, nitrate patches, isosorbide dinitrate, and isosorbide-5-mononitrate.

Calcium channel blockers prevent the normal flow of calcium into the cells of the heart and blood vessels causing the blood vessels to relax thereby increasing the supply of blood and oxygen to the heart. Examples of calcium channel blockers include amlodipine (Norvasc, Lotrel), bepridil (Vascor), diltiazem (Cardizem, Tiazac), felodipine (Plendil), nifedipine (Adalat, Procardia), nimodipine (Nimotop), nisoldipine (Sular), verapamil (Calan, Isoptin, Verelan), and nicardipine.

### Heart Failure Agents

Agents used to treat heart failure include diuretics, ACE inhibitors, vasodilators, and cardiac glycosides. Diuretics eliminate excess fluids in the tissues and circulation thereby relieving many of the symptoms of heart failure. Examples of diuretics include hydrochlorothiazide, metolazone (Zaroxolyn), furosemide (Lasix), bumetanide (Bumex), spironolactone (Aldactone), and eplerenone (Inspra).

Angiotensin converting enzyme (ACE) inhibitors reduce the workload on the heart by expanding the blood vessels and decreasing resistance to blood flow. Examples of ACE inhibitors include benazepril (Lotensin), captopril (Capoten), enalapril (Vasotec), fosinopril (Monopril), lisinopril (Prinivil, Zestril), moexipril (Univasc), perindopril (Aceon), quinapril (Accupril), ramipril (Altace), and trandolapril (Mavik).

Vasodilators reduce pressure on the blood vessels by making them relax and expand. Examples of vasodilators include hydralazine, diazoxide, prazosin, clonidine, and methyldopa. ACE inhibitors, nitrates, potassium channel activators, and calcium channel blockers also act as vasodilators.

Cardiac glycosides are compounds that increase the force of the heart's contractions. These compounds strengthen the pumping capacity of the heart and improve irregular heartbeat activity. Examples of cardiac glycosides include digitalis, digoxin, and digitoxin.

### Antithrombotic Agents

Antithrombotics inhibit the clotting ability of the blood. There are three main types of antithrombotics - platelet inhibitors, anticoagulants, and thrombolytic agents.

Platelet inhibitors inhibit the clotting activity of platelets, thereby reducing clotting in the arteries. Examples of platelet inhibitors include acetylsalicylic acid (aspirin), ticlopidine, clopidogrel (plavix), dipyridamole, cilostazol, persantine sulfinpyrazone, dipyridamole, indomethacin, and glycoprotein llb/llla inhibitors, such as abciximab, tirofiban, and eptifibatide (Integrelin). Beta blockers and calcium channel blockers also have a platelet-inhibiting effect.

Anticoagulants prevent blood clots from growing larger and prevent the formation of new clots. Examples of anticoagulants include bivalirudin (Angiomax), warfarin (Coumadin), unfractionated heparin, low molecular weight heparin, danaparoid, lepirudin, and argatroban.

Thrombolytic agents act to break down an existing blood clot. Examples of thrombolytic agents include streptokinase, urokinase, and tenecteplase (TNK), and tissue plasminogen activator (t-PA).

### Antiarrhythmic agents

Antiarrhythmic agents are used to treat disorders of the heart rate and rhythm. Examples of antiarrhythmic agents include amiodarone, quinidine, procainamide, lidocaine, and propafenone. Cardiac glycosides and beta blockers are also used as antiarrhythmic agents.

### Antihypertensive agents

Antihypertensive agents are used to treat hypertension, a condition in which the blood pressure is consistently higher than normal. Hypertension is associated with many aspects of cardiovascular disease, including congestive heart failure, atherosclerosis, and clot formation. Examples of antihypertensive agents include alpha-1-adrenergic antagonists, such as prazosin (Minipress), doxazosin mesylate (Cardura), prazosin hydrochloride (Minipress), prazosin, polythiazide (Minizide), and terazosin hydrochloride (Hytrin); beta-adrenergic antagonists, such as propranolol (Inderal), nadolol (Corgard), timolol (Blocadren), metoprolol (Lopressor), and pindolol (Visken); central alpha-adrenoceptor agonists, such as clonidine hydrochloride (Catapres), clonidine hydrochloride and chlorthalidone (Clorpres, Combipres), guanabenz Acetate (Wytensin), guanfacine hydrochloride (Tenex), methyldopa (Aldomet), methyldopa and chlorothiazide (Aldoclor), methyldopa and hydrochlorothiazide (Aldoril); combined alpha/beta-adrenergic antagonists, such as labetalol (Normodyne, Trandate), Carvedilol (Coreg); adrenergic neuron blocking agents, such as guanethidine (Ismelin), reserpine (Serpasil); central nervous system-acting antihypertensives, such as clonidine (Catapres), methyldopa (Aldomet), guanabenz (Wytensin); anti-angiotensin II agents; ACE inhibitors, such as perindopril (Aceon) captopril (Capoten), enalapril (Vasotec), lisinopril (Prinivil, Zestril); angiotensin-II receptor antagonists, such as Candesartan (Atacand), Eprosartan (Teveten), Irbesartan (Avapro), Losartan (Cozaar), Telmisartan (Micardis), Valsartan (Diovan); calcium channel blockers, such as verapamil (Calan, Isoptin), diltiazem (Cardizem), nifedipine (Adalat, Procardia); diuretics; direct vasodilators, such as nitroprusside (Nipride), diazoxide (Hyperstat IV), hydralazine (Apresoline), minoxidil (Loniten), verapamil; and potassium channel activators, such as aprikalim, bimakalim, cromakalim, emakalim, nicorandil, and pinacidil.

### Lipid Lowering Agents

Lipid lowering agents are used to lower the amounts of cholesterol or fatty sugars present in the blood. Examples of lipid lowering agents include bezafibrate (Bezalip), ciprofibrate (Modalim), and statins, such as atorvastatin (Lipitor), fluvastatin (Lescol), lovastatin (Mevacor, Altocor), mevastatin, pitavastatin (Livalo, Pitava) pravastatin (Lipostat), rosuvastatin (Crestor), and simvastatin (Zocor).

In this invention, the patient presenting with an acute coronary disease event often suffers from secondary medical conditions such as one or more of a metabolic disorder, a pulmonary disorder, a peripheral vascular disorder, or a gastrointestinal disorder. Such patients can benefit from treatment of a combination therapy comprising administering to the patient ranolazine in combination with at least one therapeutic agent.

### Pulmonary Disorders

Pulmonary disorder refers to any disease or condition related to the lungs. Examples of pulmonary disorders include, without limitation, asthma, chronic obstructive pulmonary disease (COPD), bronchitis, and emphysema.

Examples of therapeutics agents used to treat pulmonary disorders include bronchodilators including beta2 agonists and anticholinergics, corticosteroids, and electrolyte supplements. Specific examples of therapeutic agents used to treat pulmonary disorders include epinephrine, terbutaline (Brethaire, Bricanyl), albuterol (Proventil), salmeterol (Serevent, Serevent Diskus), theophylline, ipratropium bromide (Atrovent), tiotropium (Spiriva), methylprednisolone (Solu-Medrol, Medrol), magnesium, and potassium.

### Metabolic Disorders

Examples of metabolic disorders include, without limitation, diabetes, including type I and type II diabetes, metabolic syndrome, dyslipidemia, obesity, glucose intolerance, hypertension, elevated serum cholesterol, and elevated triglycerides.

Examples of therapeutic agents used to treat metabolic disorders include antihypertensive agents and lipid lowering agents, as described in the section "Cardiovascular Agent Combination Therapy" above. Additional therapeutic agents used to treat metabolic disorders include insulin, sulfonylureas, biguanides, alpha-glucosidase inhibitors, and incretin mimetics.

### Peripheral Vascular Disorders

Peripheral vascular disorders are disorders related to the blood vessels (arteries and veins) located outside the heart and brain, including, for example peripheral arterial disease (PAD), a condition that develops when the arteries that supply blood to the internal organs, arms, and legs become completely or partially blocked as a result of atherosclerosis.

### Gastrointestinal Disorders

Gastrointestinal disorders refer to diseases and conditions associated with the gastrointestinal tract. Examples of gastrointestinal disorders include gastroesophageal reflux disease (GERD), inflammatory bowel disease (IBD), gastroenteritis, gastritis and peptic ulcer disease, and pancreatitis.

Examples of therapeutic agents used to treat gastrointestinal disorders include proton pump inhibitors, such as pantoprazole (Protonix), lansoprazole (Prevacid), esomeprazole (Nexium), omeprazole (Prilosec), rabeprazole; H2 blockers, such as cimetidine (Tagamet), ranitidine (Zantac), famotidine (Pepcid), nizatidine (Axid); prostaglandins, such as misoprostoL (Cytotec); sucralfate; and antacids.

### Antibiotics, analgesics, antidepressants and anti-anxiety agents

Patients presenting with an acute coronary disease event may exhibit conditions that benefit from administration of therapeutic agent or agents that are antibiotics, analgesics, antidepressant and anti-anxiety agents in combination with ranolazine.

### Antibiotics

Antibiotics are therapeutic agents that kill, or stop the growth of, microorganisms, including both bacteria and fungi. Example of antibiotic agents include β-Lactam antibiotics, including penicillins (amoxicillin), cephalosporins, such as cefazolin, cefuroxime, cefadroxil (Duricef), cephalexin (Keflex), cephradine (Velosef), cefaclor (Ceclor), cefuroxime axtel (Ceftin), cefprozil (Cefzil), loracarbef (Lorabid), cefixime (Suprax), cefpodoxime proxetil (Vantin), ceftibuten (Cedax), cefdinir (Omnicef), ceftriaxone (Rocephin), carbapenems, and monobactams; tetracyclines, such as tetracycline; macrolide antibiotics, such as erythromycin; aminoglycosides, such as gentamicin, tobramycin, amikacin; quinolones such as ciprofloxacin; cyclic peptides, such as vancomycin, streptogramins, polymyxins; lincosamides, such as clindamycin; oxazolidinoes, such as linezolid; and sulfa antibiotics, such as sulfisoxazole.

### Analgesics

Analgesics are therapeutic agents that are used to relieve pain. Examples of analgesics include opiates and morphinomimetics, such as fentanyl and morphine; paracetamol; NSAIDs, and COX-2 inhibitors.

### Antidepressant and Anti-anxiety agents

Antidepressant and anti-anxiety agents include those agents used to treat anxiety disorders, depression, and those used as sedatives and tranquillers. Examples of antidepressant and anti-anxiety agents include benzodiazepines, such as diazepam, lorazepam, and midazolam; enzodiazepines; barbiturates; glutethimide; chloral hydrate; meprobamate; sertraline (Zoloft, Lustral, Apo-Sertral, Asentra, Gladem, Serlift, Stimuloton); escitalopram (Lexapro, Cipralex); fluoxetine (Prozac, Sarafem, Fluctin, Fontex, Prodep, Fludep, Lovan); venlafaxine (Effexor XR, Efexor); citalopram (Celexa, Cipramil, Talohexane); paroxetine (Paxil, Seroxat, Aropax); trazodone (Desyrel); amitriptyline (Elavil); and bupropion (Wellbutrin, Zyban).

Accordingly, one aspect of the invention provides for a composition comprising ranolazine and at least one therapeutic agent. In an alternative embodiment, the composition comprises ranolazine and at least two therapeutic agents. In further alternative embodiments, the composition comprises ranolazine and at least three therapeutic agents, ranolazine and at least four therapeutic agents, or ranolazine and at least five therapeutic agents.

Another aspect of the invention provides a method for treating a patient suffering from an acute cardiovascular disease event and at least one other disease or condition, which method comprises administering to the patient ranolazine in combination with at least one therapeutic agent. In an alternative embodiment, the invention provides a method for treating a patient suffering from an acute cardiovascular disease event and at least two other diseases or conditions, the method comprising administering to the patient ranolazine in combination with at least two therapeutic agents. In a further alternative embodiment, the invention provides for a method for treating a patient suffering from an acute cardiovascular disease event and at least three other diseases or conditions, the method comprising administering to the patient ranolazine in combination with at least three therapeutic agents. In a further alternative embodiment, the invention provides a method for treating a patient suffering from an acute cardiovascular disease event and at least four diseases or conditions, the method comprising administering to the patient ranolazine in combination with at least four therapeutic agents. In yet a further alternative embodiment, the invention provides a method for treating a patient suffering from an acute cardiovascular disease event and at least five diseases or conditions, the method comprising administering to the patient ranolazine in combination with at least five therapeutic agents.

The methods of combination therapy include co-administration of a single formulation containing the ranolazine and therapeutic agent or agents, essentially contemporaneous administration of more than one formulation comprising the ranolazine and therapeutic agent or agents, and consecutive administration of ranolazine and therapeutic agent or agents, in any order, wherein preferably there is a time period where the ranolazine and therapeutic agent or agents simultaneously exert their therapeutic affect. Preferably the ranolazine is administered in an IV formulation as described herein.

The following Examples are representative of the invention, but are not to be construed as limiting the scope of the claims.

### EXAMPLE 1

### Background

Recurrent ischemia after admission for non-STE acute coronary syndrome (NSTEACS) is common, though its relationship to short and long term cardiovascular outcomes in contemporary practice is not well defined. Continuous ECG (CECG) monitoring is a sensitive marker of recurrent ischemia.

### Methods

Between Oct 2004 and May 2006, 6560 patients admitted with NSTEACS were randomized to the novel anti-ischemic agent ranolazine or placebo. At randomization, 3-lead CECG monitoring was initiated (median duration 6.9 days). Recurrent ischemia was defined as ST dep ≥1 mm from baseline lasting ≥1 min. At the time of submission, data for 3479 patients was available. Median clinical follow-up was 436 days.

### Results

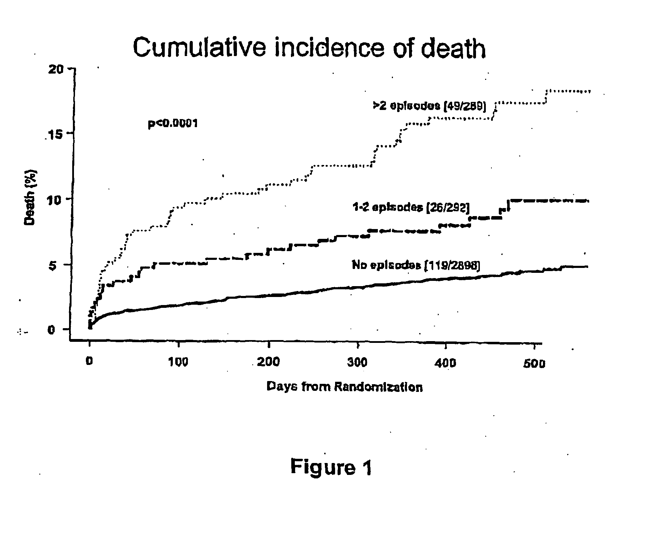

Recurrent ischemia was detected in 581/3479 patients (16.7%) with 289 patients (8.3%) experiencing >2 episodes. Among patients with an ischemic episode, the median total duration of ischemia was 263 minutes. Compared to patients with no ischemia, patients with ST depression had higher rates of death/myocardial infarction (MI) (25.7% v. 16% v. 8.6% for patients with >2, 1-2, and no episodes, respectively, p<0.0001) and death alone (see Figure 1) with a similar pattern when excluding patients with an event in the first 7 days after randomization (p<0.0001). Elderly patients (>75yrs) were more likely to have ischemia on CECG (24.7 v. 15.3%, p<0.05) but there was no difference in the rates according to index diagnosis (18.1% for NSTEMI v. 15.5% for UA).

### Conclusion

In this large study of continuous ischemic monitoring in patients with NSTEACS, ischemia was strongly associated with short and long-term mortality and MI.

### EXAMPLE 2

20-mL Type 1 flint vial of Ranolazine Injection filled to deliver 20 mL (at 1, 5, or 25 mg/mL ranolazine concentration).
Compositions:

| | |
|---|---|
| Ranolazine | 1.0, 5.0, 25.0 mg/mL |
| Dextrose monohydrate | 55.0, 52.0, 36.0 mg/mL |
| Hydrochloric acid | q.s. pH to 4.0 ± 0.2 |
| Sodium hydroxide | q.s. pH to 4.0 ± 0.2 |
| Water for Injection q.s. | |

Container/Closure System:
Vial: Type 1 Flint, 20-cc, 20-mm finish
Stopper: Rubber, 20-mm, West 4432/50, gray butyl, teflon coated
Seal: Aluminum, 20-mm, flip-top oversea

### Method of Manufacture

The intraveous formulation of ranolazine is manufactured via an aseptic fill process as follows. In a suitable vessel, the required amount of dextrose monohydrate was dissolved in Water for Injection (WFI) at about 78% of the final batch weight. With continuous stirring, the required amount of ranolazine was added to the dextrose solution. To facilitate the dissolution of ranolazine, the solution pH was adjusted to a target of 3.88-3.92 with an 0.1 N or 1.0 N HCl solution. Additionally, 1 N NaOH may have been utilized to further adjust the solution to the target pH of 3.88-3.92. After ranolazine was dissolved, the batch was adjusted to the final weight with WFI. Upon confirmation that in-process specifications had been met, the ranolazine-formulated bulk solution was sterilized by sterile filtration through two 0.2 µm sterile filters. Subsequently, the sterile ranolazine-formulated bulk solution was aseptically filled into sterile glass vials and aseptically stoppered with sterile stoppers. The stoppered vials were then sealed with clean flip-top aluminum overseals. The vials then went through a final inspection.

### EXAMPLE 3

20-mL Type I flint vial of Ranolazine Injection are filled to deliver 20 mL (25 mg/mL concentration).
Composition:

| | |
|---|---|
| Ranolazine | 25.0 mg/mL |
| Dextrose monohydrate | 36.0 mg/mL |
| Hydrochloric acid | Adjust pH to 3.3-4.7 |
| Water for Injection | q.s. |
| Container/Closure System: | |
| Vial: | Type 1 tubing, untreated, 20-mL, 20-mm finish |
| Stopper: | Rubber, 20-mm, West 4432/50, gray butyl |
| Seal: | Aluminum, 20-mm, blue flip-off overseal |

### Method of Manufacture

Water for Injection (WFI) is charged in a suitable vessel at about 90% of the final batch weight. About 90-95% of the required amount of 5 N HCl is added into the compounding vessel. With continuous stirring, the required amount of ranolazine is slowly added, followed by the addition of dextrose monohydrate into the ranolazine solution. To solubilize ranolazine, the solution pH is adjusted with 5 N HCl solution to a target of 3.9-4.1. The batch is subsequently adjusted to the final weight with WFI. Upon confirmation that in-process specifications have been met, the ranolazine-formulated bulk solution is sterilized by filtration through two redundant 0.22 µm sterilizing filters. The sterile ranolazine-formulated bulk solution is then aseptically filled into 20 mL sterile/depyrogenated vials and aseptically stoppered with sterile/depyrogenated stoppers. The stoppered vials are sealed with clean flip-top aluminum overseas. The sealed vials are terminally sterilized by a validated terminal sterilization cycle at 121.1°C for 30 minutes. After the terminal sterilization process, the vials go through an inspection. To protect the drug product from light, the vials are individually packaged into carton boxes.

### EXAMPLE 4

### Patients with Diabetes or the Metabolic Syndrome Presenting with non-ST-Elevation Acute Coronary Syndrome (NSTEACS)

### Background

Data obtained from a clinical trial of patients admitted with non-ST elevation acute coronary syndrome (NSTEACS) was evaluated to determine the prevalence and outcome of those patients also suffering with diabetes and/or metabolic syndrome. The patients were treated with ranolazine which has been associated with improved glycemic parameters. See United States Patent Application serial number, 10/443,314, published as US 2004/0063717, incorporated by reference herein in its entirety.

### Methods

MERLIN-TIMI 36 randomized 6560 patients at presentation with NSTEACS were treated with either placebo or the anti-ischemic agent ranolazine, which has also been associated with improved glycemic parameters. Median clinical follow-up was 12 months. Metabolic syndrome was defined as having any 3 of the following: 1) waist circumference ≥102cm (men) and ≥88cm (women), 2) triglycerides (TG) ≥150 mg/dL or drug treatment for elevated TG, 3) High density lipoproteins (HDL) <40 mg/dL (men) and <50 mg/dL (women), or drug treatment for reduced HDL, 4) Systolic blood pressure (SBP) ≥130 mmHg or diastolic blood pressure (DBP) ≥85 mmHg or drug treatment for hypertension, and 5) fasting glucose >100 mg/dL.

### Results

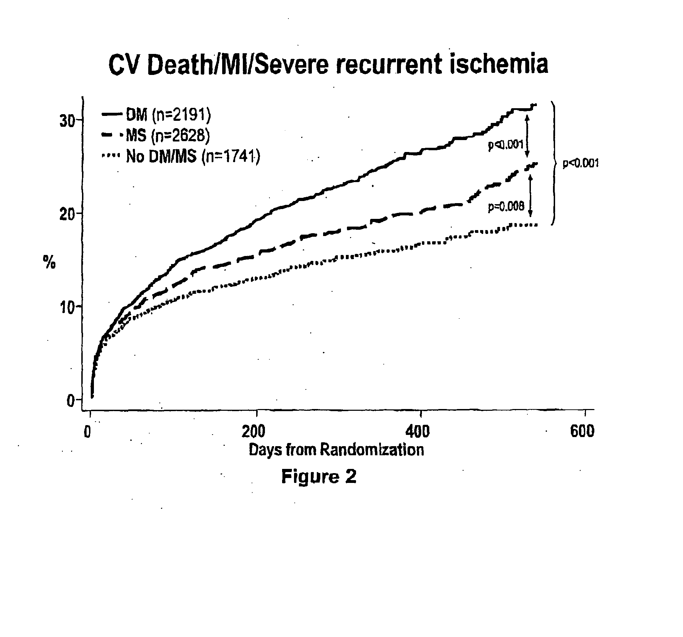

At randomization, 2191(33.4%) of all patient carried a diagnosis of diabetes mellitus (DM) and 2628 (40.1%) patients had metabolic syndrome. Patients with DM and metabolic syndrome were more likely to be female and have known coronary artery disease and had higher TIMI Risk scores at presentation, but were less likely to have an index diagnosis of NSTEMI (44.8% for DM v. 51.2% for metabolic syndrome v. 62.8% for no diagnosis, p<0.001). The rate of revascularization was similar among all groups (40.4% v. 39.7% v. 37.4%, p=0.11). There was a stepwise increase in the risk of severe recurrent ischemia, myocardial infarction, and cardiovascular death in patients with DM at highest risk followed by those with metabolic syndrome and then patients with neither at lowest risk. (Figure 2).

### Conclusions

Metabolic syndrome and diabetes are common among patients presenting with NSTEACS and confer increased cardiovascular risk.

### EXAMPLE 5

### Baseline Clinical Risk and Recurrent Ischemia as Detected on Continuous ECG (CECG) monitoring in patients admitted with non-ST-Elevation Acute Coronary Syndrome

### Background

To evaluate the association between the TIMI Risk Score (TRS) for NSTEACS and subsequent ischemia detected on Continuous ECG (CECG) monitoring and determine if ranolazine affects this relationship.

### Methods

MERLIN-TIMI 36 randomized 6560 patients at presentation with NSTEACS to the anti-ischemic agent ranolazine or placebo. Median clinical follow-up was 12 months. At randomization, 3-lead CECG monitoring was initiated for median duration of 6.9 days. Recurrent ischemia on CECG was defined as ST dep >1mm from baseline lasting >1 min. The TRS is calculated as the sum of seven presenting characteristics : 1) age >65 yrs, 2) >3 cardiac risk factors, 3) documented coronary artery disease, 4) recent severe angina, 5) ST deviation >0.5 mm, 6) elevated cardiac markers, and 7) prior aspirin use and is categorized as low (0-2), moderate (3-4), or high (>4) risk.

### Results

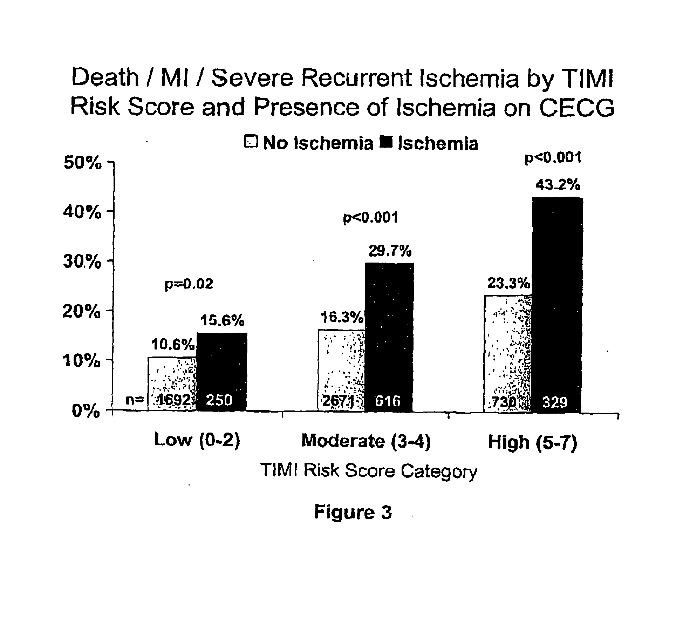

Preliminary results show that overall, 30.9% were low risk (TRS 0-2), 52.3% were moderate risk TRS (3-4), and 16.8% were high risk (TRS 5-7). Ischemia was detected on CECG in 1195/6288 (19.0%) patients with 610 (9.7%) experiencing >2 episodes. Patients with higher TRS were more likely to experience any ischemic episode (12.9% in low TRS v. 18.7 % in moderate TRS v. 31.1% in high TRS, p<0.001) and >2 episodes (5.3% v 9.9% v. 17.3%, p<0.001). See Figure 3. Among patients who experienced ischemia, those with higher TRS had a longer total duration of ischemia (63.4 v. 105 v. 118.5 min). Among each risk category, ischemia detected on CECG was associated with worse cardiovascular outcome.

### Conclusions

Recurrent ischemia as detected on CECG was more frequent among patients with higher clinical risk as determined by the TRS. Even among patients with a similar baseline TRS category, the subsequent development of recurrent ischemia on CECG was associated with worse long-term cardiovascular outcomes.

### EXAMPLE 6

### Results of MERLIN-TIMI 36

The results of the MERLIN-TIMI 36 study are presented herewith.

The MERLIN-TIMI 36 study had the enrollment seen in Table I below.

**Table 1**

| **MERLIN - TIMI 36: Enrollment** |
|---|
| • Patients |
| - 2485 Eastern Europe |
| - 2973 Western Europe (including Israel, S. Africa) |
| - 1102 North America |
| ○ 720 USA |
| ○ 382 Canada |
| • Study duration |
| - First patient randomized: October 8, 2004 |
| - Last patient completed: February 14, 2007 |
| - Mean total follow-up time |
| ○ Placebo: 347 days |
| ○ Ranolazine: 346 days |

At the end of the study the 6560 patients had been randomized and followed as shown in Table 2 below.

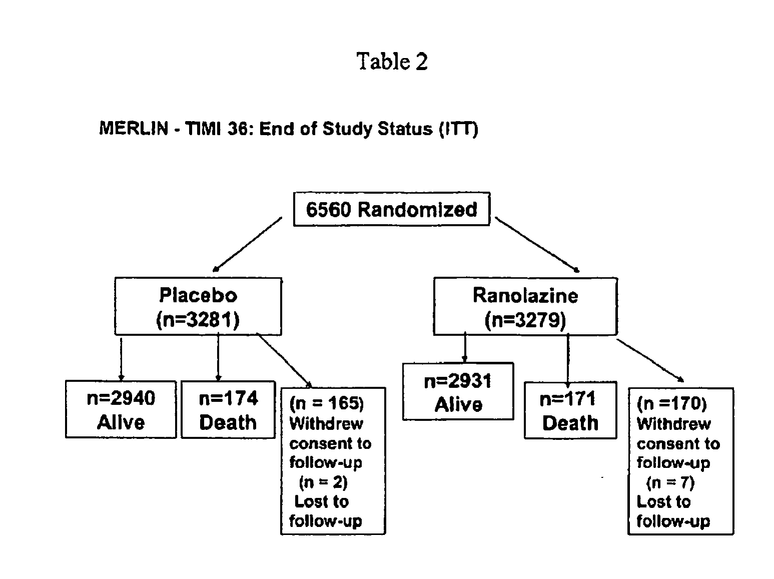

The demographic/baseline characteristics for the 6560 patients are shown in Table 3 below.

**Table 3**

| **MERLIN - TIMI 36: Demographic/Baseline Characteristics (ITT)** | | | |
|---|---|---|---|
| | | **Placebo (n=3281)** | **Ranolazine (n=3279)** |
| **Age (years)** | **Mean** | **83.6** | **63.3** |
| | | | |
| | **<65** | **1647 (50%)** | **1703 (52%)** |
| | **65-74** | **1042 (32%)** | **1014 (31%)** |
| | **>=75** | **592 (18%)** | **562 (17%)** |
| | | | |
| **Gender Race** | **Male** | **2096 (64%)** | **2173 (66%)** |
| | **Female** | **1185 (36%)** | **1106 (34%)** |
| | | | |
| | **Aslan** | **39 (1%)** | **49 (1%)** |
| | **Black** | **53 (2%)** | **50 (2%)** |
| | | | |
| | **Caucaslan** | **3128 (95%)** | **3112 (95%)** |
| | **Hispanic** | **23 (<1%)** | **23 (<1%)** |
| | **Other** | **31 (1%)** | **45 (1%)** |

| | | | |
|---|---|---|---|
| **p < 0.05** | | | |

The cardiovascular history of the MERLIN-TIMI 36 patients is in Tables 4, 5, and 6 below.

**Table 4**

| **MERLIN - TIMI 36: CV History (ITT)** | | |
|---|---|---|
| | **Placebo (n=3281)** | **Ranolazine (n=3279)** |
| **MI** | **1095 (33%)** | **1119 (34%)** |
| **Unstable angina** | **908 (28%)** | **892 (27%)** |
| **Angina pectoris CCSC 1 month prior** | **1776 (54%)** | **1789 (55%)** |
| **None** | **154 (9%)** | **195 (11%)** |
| **I** | **277 (16%)** | **296 (17%)** |
| **II** | **715 (41%)** | **743 (42%)** |
| **III** | **468 (27%)** | **421 (24%)** |
| **IV** | **136 (8%)** | **111 (6%)** |

| | | |
|---|---|---|
| ***p < 0.05** | | |

**Table 5**

| **MERLIN - TIMI 36: CV History (ITT) cont** | | |
|---|---|---|
| | **Placebo (n=3281)** | **Ranolazine (n=3279)** |
| | | |
| **Prior coronary anglography** | **1102 (34%)** | **1117 (34%)** |
| **Stenosls >=50%** | **930 (84%)** | **930 (83%)** |
| | | |
| **PCI** | **636 (19%)** | **684 (21%)** |
| | | |
| **CABG** | **380 (12%)** | **389 (12%)** |

**Table 6**

| **MERLIN - TIMI 36: CV History (ITT) cont.** | | |
|---|---|---|
| | **Placebo (n=3281)** | **Ranolazine (n=3279)** |
| **Ventricular arrhythmia** | **124 (4%)** | **119 (4%)** |
| **Hypertension** | **2409 (73%)** | **2395 (73%)** |
| **CHF** | **557 (17%)** | **538 (16%)** |
| **Current NYHA class** | | |
| **I** | **78 (14%)** | **72 (13%)** |
| **II** | **337 (61%)** | **326 (62%)** |
| **III** | **121 (22°%)** | **122 (23%)** |
| **IV** | **16 (3%)** | **9 (2%)** |
| | | |
| **PVD** | **296 (9%)** | **276 (8%)** |
| **Cerebrovascular disease** | **357 (11%)** | **356 (11%)** |

Patients in the MERLIN-TIMI 36 study were found to have the TIMI Risk Factors shown in Table 7 below.

**Table 7**

| **MERLIN - TIMI 36: Risk Factors (ITT)** | | |
|---|---|---|
| | **Placebo (n=3281)** | **Ranolazine (n=3279)** |
| **Dyslipidemia** | **2022 (62%)** | **2028 (62%)** |
| **Diabetes** | **1116 (34%)** | **1104 (34%)** |

| **TIMI Risk Score** | | |
|---|---|---|
| **0 - 2** | **884 (27%)** | **882 (27%)** |
| **3 - 4** | **1730 53%)** | **1727 (53%)** |
| **5 - 7** | **867 (20%)** | **670 (20%)** |

Patients in the MERLIN-TIMI 36 study were found to have the Qualifying Index Event shown in Table 8 below.

**Table 8**

| MERLIN - TIMI 36: Qualifying Index Event (ITT) | | |
|---|---|---|
| | Placebo (n=3281) | Ranolazine (n=3279) |
| Time from onset of event to randomization | | |
| 0- 24 hrs | 1677 (51%) | 1645 (50%) |
| >24 - 48 hrs | 1368 (42%) | 1403 (43%) |
| >48 hrs | 234 (7%) | 231 (7%) |
| Time from hospitalization for event to randomization | | |
| 0 - 24 hrs | 1886 (61%) | 2007 (61%) |
| >24 - 48 hrs | 986 (30%) | 962 (29%) |
| >48 hrs | 304 (9%) | 310 (9%) |
| Duration of Index event (hrs) - Mean (SD) | 2.6 (5.3) | 2.7 (5.9) |
| Confirmed diagnosis | | |
| Non-STE MI | 1667 (51%) | 1875 (51%) |
| Unstable angina | 1526 (47%) | 1541 (47%) |
| Other | 88 (2%) | 63 (2%) |

Patients in the MERLIN-TIMI 36 study were given IV, IV + oral, or oral dosages of either placebo or ranolazine at the IV rates or final oral dose (mg) as shown in Table 9 below.

**Table 9**

| MERLIN - TIMI 36: Study Drug Exposure and Administration | | |
|---|---|---|
| | Placebo (n=3273) | Ranolazine (n=3268) |
| | Mean (SD) | Mean (SD) |
| Hours on IV | 27 (14) | 27 (15) |
| Days on oral | 302 (166) | 284 (174) |
| Days on IV + oral | 297 (170) | 279 (177) |

| Final IV rate (mg/hr) | n (%) | n (%) |
|---|---|---|
| 80 | 3122 (95%) | 3090 (95%) |
| 60 | 37 (1%) | 51 (2%) |
| 40 | 94 (3%) | 93 (3%) |
| <40 | 6 (<1%) | 19 (<1%) |
| Other | 7 (<1%) | 7 (<1%) |

| Final oral dose (mg) | | |
|---|---|---|
| 1000 | 2910 (89%) | 2715 (83%) |
| 750 | 112 (3%) | 180 (6%) |
| 500 | 158 (5%) | 235 (7%) |
| 375 | 25 (<1%) | 64 (2%) |

The Intention to Treat (ITT) and safety analysis data for the MERLIN-TIMI 36 study is shown in Table 10 below.

**Table 10**

| Intention to Treat and Safety Analysis | | | | |
|---|---|---|---|---|
| | ITT | | Safety | |
| Randomization: | Placebo N = 3281 | Ranolazine N = 3279 | Placebo N = 3273 | Ranolazine N = 3288 |
| Received assigned treatment | N = 3273 | N = 3268 | N = 3272 | N = 3267 |
| Never treated | N = 8 | N = 11 | Excluded | Excluded |
| Received wrong treatment throughout study | N = 1 Analyzed as randomized | N = 1 Analyzed as randomized | N =1 Analyzed as treated | N = 1 Analyzed as treated |
| Received wrong treatment during part of study | None | N = 3 | None | All events attributed to ranolazine |

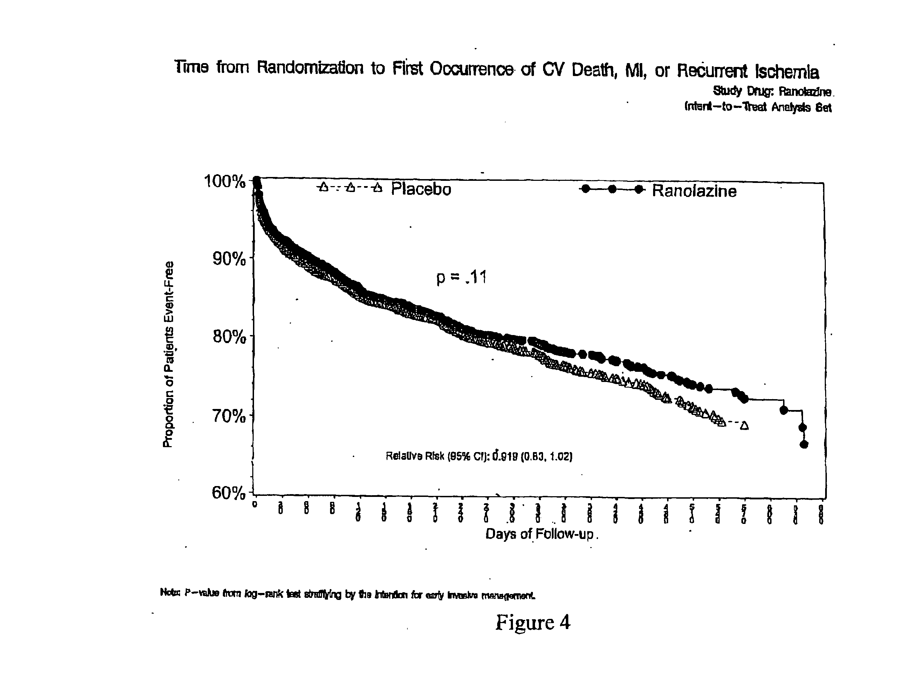

The MERLIN-TIMI 36 primary efficacy endpoint (ITT) time from randomization to first occurrence of cardiovascular death, myocardial infarction, or recurrent ischemia data is shown in Table 11 below (and Figures 4 and 5).

**Table 11**

| MERLIN - TIMI 36: Primary Efficacy Endpoint (ITT) Time from Randomization to First Occurrence of CV Death, MI, or Recurrent Ischemia | | | |
|---|---|---|---|
| | Placebo (n = 3281) | | Ranolazine (n = 3279) |
| Pts with events | 753 | | 695 |
| Endpoint-defining events | | | |
| CV death | 78 (10.4%) | | 87 (12.5%) |
| MI | 210 (27.9%) | | 208 (29.9%) |
| Rt | 465 (61.8%) | | 400 (57.6%) |
| Severe RI | 332 (44.1%) | | 302 (43.5%) |
| Worsen, angina/ischemia | 113 (17.7%) | | 98 (14.1%) |
| Relative risk (SE) | | 0.919 (.05) | |
| 95% Confidence interval | | (0.83, 1.02) | |
| Log rank test p-value | | 0.11 | |
| Incidence (Kaplan-Meler estimate) | | | |
| 30 days | 8.3% | | 7.7% |
| 60 days | 10.9% | | 9.9% |
| 380 days | 23.5°6 | | 21.8% |
| 540 days | 30.1% | | 26.3% |

The MERLIN-TIMI 36 major secondary efficacy endpoint (ITT) time from randomization to first occurrence of cardiovascular death, myocardial infarction, or severe recurrent ischemia data is shown in Table 12 below (and Figures 6 and 7).

**Table 12**

| MERLIN - TIMI 36: Major Secondary Efficacy Endpoint (ITT) Time from Randomization to First Occurrence of CV Death, MI, or Severe Recurrent Ischemia | | | |
|---|---|---|---|
| | Placebo (n = 3281) | | Ranolazine (n = 3279) |
| Pts with events | 625 | | 602 |
| Endpoint-defining events | | | |
| CV death | 79 (12.6%) | | 88 (14.6%) |
| MI | 215 (34.4%) | | 212 (35.2%) |
| Severe Pd | 331 (53.0%) | | 302 (50.2%) |
| Relative risk (SE) | | 0.962 (0.06) | |
| 95% Confidence Interval | | (0.86, 1.08) | |
| Log rank p-value | | 0.50 | |
| Incidence (Kaplan-Meler estimate) | | | |
| 30 days | 8.2% | | 7.5% |
| 60 days | 10.4% | | 9.4% |
| 360 days | 19.2% | | 18.7% |
| 540 days | 24.9% | | 22.4% |

The p values for the testing of secondary efficacy endpoints (ITT) in MERLIN-TIMI 36 are shown in Table 13 below.

**Table 13**

| MERLIN - TIMI 36: Testing of Secondary Efficacy Endpoints (ITT) | |
|---|---|
| | P - value |
| Time to CV death, MI, or RI (Primary) | 0.11 |
| Time to CV death, MI, or SRI (Major Secondary) | 0.50 |
| Time to failure of therapy (Secondary) | 0.15 |
| Incidence at 30 days CV death, MI, SRI, or + Holter for Ischemia (Secondary) | 0.055 |
| SAQ - Anginal Frequency (Secondary) | <.001 |
| SAQ-Physical Limitation (Secondary) | 0.91 |
| Duration of exercise on ETT (Secondary) | 0.35 |
| Total duration of ischemia on Holter from randomization up to 72 hrs (Secondary) | 0.26 |

| | |
|---|---|
| Note: P-value < 0.0497 required for statistical significance | |

The secondary efficacy endpoint time from randomization to failure of therapy (cardiovascular death, myocardial ischemia, recurrent ischemia, positive Holter for ischemia, New/Worsening heart failure, or early +ETT) data from MERLIN-TIMI 36 is shown in Table 14 below (and Figures 8 and 9).

**Table 14**

| MERLIN - TIMI 36: Secondary Efficacy Endpoint (ITT) Time from Randomization to Failure of Therapy (CV Death, MI, RI, + Holter for ischemia, New/Worsening HF, or Early + ETT) | | | |
|---|---|---|---|
| | Placebo (n = 3281) | | Ranolazine (n = 3279) |
| Pts with events | 1233 | | 1172 |
| Relative risk (SE) | | 0.943 (0.04) | |
| 95% Confidence Interval | | (0.87, 1.02) | |
| Log rank test p-value | | 0.18 | |
| Incidence (Kaplan-Meier estimate) | | | |
| 30 days | 25.7% | | 24.1% |
| 60 days | 27.6% | | 25.6% |
| 380 days | 38.3% | | 36.8% |
| 540 days | 43.0% | | 39.9% |

The secondary efficacy endpoint (ITT) incidence at 30 days of cardiovascular death, myocardial infarction, severe recurrent ischemia, or positive Holter for ischemia data from MERLIN-TIMI 36 is shown in Table 15 below.

**Table 15**

| MERLIN - TIMI 36: Secondary Efficacy Endpoint (ITT) Incidence at 30 days of CV Death, MI, Severe RI, or Positive Holter for Ischemia | | | |
|---|---|---|---|
| | Placebo (n = 3281) | | Ranolazine (n = 3279) |
| Pts with events | 824 (25%) | | 757 (23%) |
| Relative risk | | 0.92 | |
| 95% confidence interval | | (0.84, 1.00) | |
| P-value (Cochran-Mantel-Haenszel test) | | 0.055 | |

The MERLIN-T1MI 36 secondary efficacy endpoint (ITT) Seattle Angina Questionnaire scores at 4 months for anginal frequency scale are shown in Table 16 below, and scores for physical limitation scale are shown in Table 17 below.

**Table 16**

| MERLIN - TIMI 36: Secondary Efficacy Endpoint (ITT) Seattle Angina Questionnaire Scores at 4 Months-Anginal Frequency Scale | | | |
|---|---|---|---|
| | Placebo (n = 3281) | | Ranolazine (n = 3279) |
| N | 2884 | | 2558 |
| Mean (SEM) | 82.2 (0.4) | | 84.3 (0.4) |
| Median | 90 | | 100 |
| 25^{th} percentile - 75^{th} percentile | 70 - 100 | | 80 - 100 |
| Min- Max | 0-100 | | 0 - 100 |
| P-value (CMH row mean score test) | | < .001 | |

**Table 17**

| MERLIN - TIMI 36: Secondary Efficacy Endpoint (ITT) Seattle Angina Questionnaire Scores at 4 Months-Physical Limitation Scale | | | |
|---|---|---|---|
| | Placebo (n = 3281) | | Ranolazine (n = 3279) |
| N | 2411 | | 2339 |
| Mean (SEM) | 72.7 (0.5) | | 72.8 (0.5) |
| Median | 77.8 | | 77.8 |
| 25^{th} percentile - 75^{th} percentile | 55.6 - 94.4 | | 55.6 - 94.4 |
| Min- Max | 0 - 100 | | 0 - 100 |
| P-value (CMH row mean score test) | | 0.91 | |

The duration of exercise data on ETT (exercise treadmill time) at 8 months or final visit, if earlier from the MERLIN-TIMI 36 study are shown in Table 18 below.

**Table 18**

| MERLIN - TIMI 36: Secondary Efficacy Endpoint (ITT) Duration of Exercise on ETT at 8 Months or Final Visit, if earlier | | | |
|---|---|---|---|
| | Placebo (n = 3281) | | Ranolazine (n = 3279) |
| No. patients taking ETT | 2217 | | 2198 |
| LS mean (SEM) | 542.8 (5.5) | | 550.0 (5.5) |
| LS mean difference (SEM) | | 7.23 (7.7) | |
| 95% Confidence interval | | (-7.9 - 22.3) | |
| P-value (ANOVA) | | 0.36 | |
| Treadmill: Mean (SEM) | 597.3 (7.3) (n = 1374) | | 618.7 (7.5) (n=1329) |
| Bicycle: Mean (SEM) | 471.1 (7.6) (n=843) | | 465.1 (7.1) (n=867) |

| | | | |
|---|---|---|---|
| Note: Secondary endpoint | | | |

The data for total duration (minutes) of ischemia on Holter between randomization and 72 hours from MERLIN-TEMI 36 is shown in Table 19 below.

**Table 19**

| MERLIN - TIMI 36: Secondary Efficacy Endpoint (ITT) Total Duration (min.) of Ischemia on Holter between Randomization and 72 Hours | | | |
|---|---|---|---|
| | Placebo (n = 3281) | | Ranolazine (n = 3279) |
| No. patients with Holter | 3190 (97%) | | 3165 (97%) |
| Mean (SEM) | 28.1 (2.3) | | 31.8 (2.5) |
| Median | 0 | | 0 |
| Min - Max | (0 - 2328.0) | | (0 - 2661.5) |
| P-value (CMH row mean score) | | 0.26 | |

The data for time from randomization to first occurrence of cardiovascular death or myocardial infarction from MERLIN-TIMI 36 is shown in Table 20 below. For relative risk of CV death, MI, or recurrent ischemia by subgroup, see Figure 10. For relative risk of CV death, MI, or severe recurrent ischemia by subgroup, see Figure 11. For relative risks of failure of therapy by subgroup, see Figure 12.

**Table 20**

| MERLIN - TIMI 36: Additional Efficacy Endpoint (ITT) Time from Randomization to First Occurrence of CV Death or MI | | | |
|---|---|---|---|
| | Placebo (n = 3281) | | Ranolazine (n = 3279) |
| Pts with events | | 343 | 338 |
| Endpoint-defining events | | | |
| CV death | 101 (29.4%) | | 103 (30.5%) |
| MI | 242 (70.6%) | | 235 (69.5%) |
| Relative risk (SE) | | 0.987 (0.08) | |
| 95% Confidence interval | | (0.85, 1.15) | |
| Log rank test p-value | | 0.87 | |
| Incidence (Kaplan-Meler estimate) | | | |
| 30 days | 4.7% | | 4.2% |
| 60 days | 5.8% | | 5.3% |
| 360 days | 10.5% | | 10.4% |
| 540 days | 13.6% | | 12.9% |

The safety endpoint data for time from randomization to death from any cause from MERLIN-TIMI 36 is shown in Table 21 below (see figure 13 for time from randomization to all-cause mortality, and figure 14 for cumulative hazard rates for all-cause mortality).

**Table 21**

| MERLIN - TIMI 36: Safety Endpoint (All Patients Dosed) Time from Randomization to Death from Any Cause | | | |
|---|---|---|---|
| | Placebo (n = 3273) | | Ranolazine (n = 3268) |
| Pts with events | 175 | | 172 |
| Relative risk (SE) | | 0.988 (0.11) | |
| 95% Confidence Interval | | (0.80, 1.22) | |
| Log rank test p-value | | 0.91 | |
| Incidence (Keplan-Meier estimate) | | | |
| 30 days | 1.6% | | 1.8% |
| 60 days | 2.3% | | 2.2% |
| 180 days | 3.5% | | 3.4% |
| 360 days | 5.1% | | 5.3% |
| 540 days | 7.4% | | 7.0% |

The safety endpoint data for incidence of symptomatic documented arrhythmias from MERLIN-TIMI 36 is shown in Table 22 below.

**Table 22**

| MERLIN - TIMI 36: Safety Endpoint (All Patients Dosed) Incidence of Symptomatic Documented Arrhythmias | | | |
|---|---|---|---|
| | Placebo (n = 3273) | | Ranolazine (n = 3268) |
| Pts, with Arrhythmias - Overall | 102 (3.1%) | | 99 (3.0%) |
| Ventricular arrhythmias | 29 (0.9%) | | 22 (0.7%) |
| Supraventricular arrhythmias | 41(1.3%) | | 38 (1.2%) |
| Bradyarrhythmias | 29 (0.9%) | | 34 (1.0%) |
| Cardiac arrest NOS | 9 (0.3%) | | 10 (0.3%) |
| | | | |
| Cochran-Mantel-Maenszel test p-value (overall) | | 0.84 | |

The safety endpoint data for time from randomization to first occurrence of death or cardiovascular hospitalization (all patients dosed) from MERLIN-TIMI 36 is shown in Table 23 below.

**Table 23**

| MERLIN - TIMI 36: Safety Endpoint (All Patients Dosed) Time from Randomization to First Occurrence of Death or CV Hospitalization | | | |
|---|---|---|---|
| | Placebo (n = 3273) | | Ranolazine (n = 3268) |
| Pts with events | | 1065 | 1037 |
| Endpoint-defining events | | | |
| Death | 110 (10.3%) | | 111 (10.7%) |
| CV hospitalization | 955 (89.7%) | | 926 (89.3%) |
| Relative risk (SE) | | 0.981 (0.04) | |
| 95% Confidence Interval | | (0.90, 1.07) | |
| Log rank test p-value | | 0.67 | |
| Incidence (Kaplan-Meler estimate) | | | |
| 30 days | 9.1% | | 9.2% |
| 60 days | 14.7% | | 15.0% |
| 360 days | 32.8% | | 32.8% |
| 540 days | 40.0% | | 37.6% |

The safety endpoint data for incidence of clinically significant arrhythmias during 7 day Holter monitoring from MERLIN-TIMI 36 is shown in Table 24 below.

**Table 24**

| MERLIN - TIMI 36: Safety Endpoint (All Patients Dosed) Incidence of Clinically Significant Arrhythmias during 7 Day Holter | | | |
|---|---|---|---|
| | Placebo (n = 3273) | | Ranolazine (n = 3268) |
| Any Clinically Significant Arrhythmia | 2650 (81.0%) | | 2330 (71.3%) |
| Ventricular tachycardia- | 1211 (37.0%) | | 948 (29.0%) |
| Supraventricular tachycardia^{b} | 1752 (53.5%) | | 1413 (43.2%) |
| New onset atrial fibrillation | 75 (2.3%) | | 55 (1.7%) |
| Bradycardie episode | 1485 (45.4%) | | 1257 (38.5%) |
| | | | |
| Cochran-Mantel-Haenszel test p-value (overall incidence) | | < .001 | |

| | | | |
|---|---|---|---|
| ^{a}Any VT >= 100 bpm >=3 beats ^{b}Any SVT >=120 bpm | | | |

The overview of adverse events (safety - all patients dosed) from MERLIN-TIMI 36 is shown in Table 25 below.

**Table 25**

| MERLIN - TIMI 36: Overview of Adverse Events (Safety - All Patients Dosed) | | |
|---|---|---|
| | Placebo (n=3273) | Ranolazine (n=3268) |
| Patients with AEs | 2404 (73%) | 2473 (78%) |
| Patients with study drug-related AEs | 675(21%) | 892 (30%) |
| Patients early prematurely withdrawn due to AEs | 256 (8%) | 437 (13%) |
| Patients with serious AEs (SAEs) | 1123 (34%) | 1126 (34%) |
| Patients with AEs leading to death | 175 (5%) | 172 (5%) |

The data (safety -all patients dosed) for adverse events summary (> 4% incidence) from MERLIN-TIMI 36 is shown in Table 26 below.

**Table 26**

| MERLIN - TIMI 36: Adverse Events Summary > 4% Incidence (Safety - All Patients Dosed) | | | |
|---|---|---|---|
| Body System | Preferred Term | Placebo (n=3273) | Ranolazine (n=3268) |
| Cardiac disorders | Angina unstable | 372 (11%) | 332 (10%) |
| | Angina pectoris | 256 (8%) | 258 (8%) |
| | Cardiac failure | 173 (5%) | 156 (5%) |
| GI disorders | Nausea | 196 (6%) | 302 (9%) |
| | Constipation | 114 (3%) | 297 (9%) |
| General disorders | Chest pain | 433 (13%) | 356 (11%) |
| | Asthenia | 88 (3%) | 158 (5%) |
| | Fatigue | 89 (3%) | 139 (4%) |
| Nervous system disorders | Dizziness | 224 (7%) | 411 (13%) |
| | Headache | 295 (9%) | 238 (7%) |
| Vascular disorders | Hypotension | 104 (3%) | 160 (5%) |

The data (safety - all patients dosed) for serious adverse events (≥1% incidence) from MERLIN-TIMI 36 is shown in Table 27 below.

**Table 27**

| MERLIN - TIMI 36: Serious Adverse Events ≥1% Incidence (Safety - All Patients Dosed) | | | |
|---|---|---|---|
| Body System | Preferred Term | Placebo (n=3273) | Ranolazine (n=3268) |
| Cardiac disorders | Angina unstable | 304 (9%) | 258 (8%) |
| | Cardiac failure | 146 (4%) | 126 (4%) |
| | MI | 134 (4%) | 123 (4%) |
| | Angina pectoris | 95 (3%) | 115 (4%) |
| | Acute Ml | 98 (3%) | 100 (3%) |
| | ACS | 52 (2%) | 59 (2%) |
| | Cardiac failure congestive | 34 (1%) | 47 (1%) |
| | Atrial fibrillation | 31 (<1%) | 33 (1%) |
| General disorders | Chest pain | 105 (3%) | 77 (2%) |
| Nervous system disorders | Syncope | 23 (<1%) | 34 (1%) |

The proportion of patients in the MERLIN-TIMI 36 study with HbA1C ≥7% is shown in Table 28 below (and see figure 15).

**Table 28**

| MERLIN - TIMI 36: Proportion of Patents with HbA1C ≥ 7% (Safety - All Patients Dosed) | | |
|---|---|---|
| Visit | Placebo (n=3273) | Ranolazine (n=3268) |
| Baseline | 607 (25%) | 655 (24%) |
| Month 4 | 542 (22%) | 404 (17%) |
| Month 8 | 439 (22%) | 335 (18%) |
| Month 16 | 87 (20%) | 84 (20%) |
| Final Visit | 514 (21%) | 416 (17%) |

| | | |
|---|---|---|
| Note: Baseline is In-Hospital visit prior to randomization | | |

The proportion of patients with HbA1C ≥7% (with diabetes or no diabetes) at various times during the MERLIN-TIMI 36 study is shown in Table 29 below (and figure 16).

**Table 29**

| MERLIN - TIMI 36: Proportion of Patents with HbA1C ≥ 7% by Diabetes at Enrollment (Safety - All Patients Dosed) | | | | |
|---|---|---|---|---|
| | Diabetes | | No Diabetes | |
| Visit | Placebo (n=1117) | Ranolazine (n=1098) | Placebo (n=2156) | Rarolazine (n=2170) |
| Baseline | 559 (56%) | 542 (57%) | 128 (7%) | 113 (6%) |
| Month 4 | 424 (51%) | 326 (41%) | 118 (7%) | 78 (5%) |
| Month 8 | 339 (52%) | 263 (44%) | 100 (8%) | 72 (6%) |
| Month 16 | 70 (53%) | 63 (48%) | 17 (6%) | 21 (7%) |
| Final Visit | 413 (50%) | 324 (42%) | 101 (6%) | 92 (6%) |

| | | | | |
|---|---|---|---|---|
| Note: Baseline is In-Hospital visit prior to randomization | | | | |

It is contemplated that the study shows that patients with a cardiac condition, including angina, and also a glycosylated hemoglobin level of greater than or equal to 7% responded positively to both conditions (i.e., the cardiac condition and diabetes) upon administration of ranolazine.

### EXAMPLE 7

### Effects of a Novel Anti-ischemic Agent, Ranolazine, on Recurrent Cardiovascular Events in Patients with non-ST Elevation Acute Coronary Syndromes

### Background

To determine the efficacy and safety of ranolazine during long-term treatment of patients with non-ST elevation ACS receiving standard therapy. Non-ST elevation acute coronary syndrome (ACS) is a heterogeneous condition with multiple possible etiologies that may contribute to an imbalance in myocardial oxygen supply and demand, resulting in disruption of cellular homeostasis and depletion of myocardial cellular energy stores. Despite advances in anti-thrombotic therapy, coronary revascularization, and other preventative therapies, the risk of recurrent events in this population remains substantial, in particular among those with indicators of higher risk such as diabetes mellitus, ST-segment depression, or a high TIMI Risk Score.

### Methods

Between October 2004 and May 2006, 6560 patients admitted with NSTEACS were randomized to the novel anti-ischemic agent ranolazine or placebo. At randomization, patients were assigned in a 1:1 ratio to receive either ranolazine or placebo (see Figure 17 ). Intravenous delivery of drug was initiated and administered as 200 mg intravenously over 1 hour, followed by an 80 mg/hr intravenous infusion, that was reduced to 40 mg/hr for patients with an estimated creatinine clearance <30 ml/min, and was continued for 12 to 96 hours. Upon completion of the infusion, study medication (ranolazine ER or matched placebo) was continued orally at a dose of 1000 mg BID until the end of the study (about 12 months).

### Results

The primary endpoint occurred in 696 (21.8%) of patients in the ranolazine group and 753 (23.5%) of patients in the placebo group (HR 0.92; 95% CI 0.83-1.02, P=0.11). The composite of cardiovascular death or MI occurred in 338 (10.4%) patients allocated to ranolazine and 343 (10.5%) patients allocated to placebo (HR 0.99; 95% CI 0.85-1.15, P=0.87). (See Figure 18, Kaplan-Meier Estimated Rates of the Primary Endpoint [Figure 18A, cardiovascular death, MI, or recurrent ischemia], cardiovascular death or MI [Figure 18B], and recurrent ischemia [Figure 18C]. Recurrent ischemia was significantly reduced in the ranolazine (13.9%) compared with the placebo group (16.1%; HR 0.87; 95% CI 0.76-0.99, P=0.030). Symptomatic documented arrhythmias did not differ between the ranolazine (3.0%) and placebo (3.1%) groups (P=0.84). There was no difference in total mortality with ranolazine compared with placebo (HR 0.99; 95% CI 0.80-1.22; P=0.91) (see Figure 19, Kaplan-Meier Estimated Event Rates (12 months) and Hazard Ratios for the Primary End Point in the Ranolazine Group, as compared with the Placebo Group in Various Subgroups. Those subgroups denoted with an asterix were significant at the p <0.0497 level. However, none show definitive evidence of a statistical interaction.).

### Conclusion

The addition of ranolazine to standard treatment for ACS was not significantly effective in reducing major cardiovascular events. The observed reduction in recurrent ischemia with favorable overall safety in this broad population with established coronary artery disease provides additional evidence to guide the use of ranolazine as an antianginal therapy. Ranolazine was associated with a significant reduction in the frequency of arrhythmias detected by Holter recording during the first 7 days after randomization.

Further findings from the MERLIN-TIMI 36 study are presented herewith in Appendix B, which is incorporated herein by reference in its entirety.

### EXAMPLE 8

### The Effect of Ranolazine, a Novel Anti-anginal Agent with Electrophysiologic Properties, on the Incidence of Tachyarrhythmias: Results from the MERLIN-TIMI 36 Randomized Controlled Trial

### Background

Ranolazine reduces ischemia in patients with coronary artery disease by a novel mechanism proposed to be via inhibition of the late phase of the inward sodium current during cardiac repolarization, with a consequent reduction in intracellular sodium and calcium overload. Increased intracellular calcium leads to both mechanical and electrical myocyte excitability. Despite prolonging the QTc interval (2-5 ms), ranolazine reduces pro-arrhythmic substrate such as *early afler-depolarizations* in animal models. However, the potential anti-arrhythmic actions of ranolazine had yet to be evaluated in humans.

### Methods

The MERLIN-TIMI 36 trial randomized 6560 patients hospitalized with a non-ST elevation acute coronary syndrome to ranolazine or placebo in addition to standard medical therapy. Continuous ECG (Holter) recording was performed for the first 7 days after randomization. Because of the known prolongation of the QT interval with ranolazine, analyses of a prespecified set of arrhythmias were a major objective of the trial. All arrhythmias were evaluated in the TIMI ECG Core Laboratory by cardiologists blinded to treatment and outcomes.

### Results

Of the 6560 patients in MERLIN-TIMI 36, 6351 patients (97%) had a Holter recording valid for arrhythmia analysis. Treatment with ranolazine resulted in significantly lower rates of tachyarrhythmias compared to placebo (as shown in the top half of the below table).

| | Placebo n=3189(%) | Ranolazine n=3162(%) | P value |
|---|---|---|---|
| Clinically Significant Arrhythmia on Holter | 2650 (81.0) | 2330 (71.3) | <0.001 |
| Any ventricular tachycardia >=3 beats | 1211 (37.0) | 948 (29.0) | <0.001 |
| Triplets | 1771(54.1) | 1532 (46.9) | <0.001 |
| VT >= 4 beats | 1031 (31.5) | 772 (23.6) | <0.001 |
| VT >= 8 beats (< 30 sees) | 277 (8.7) | 175 (5.5) | <0.001 |
| Polymorphic VT >= 8 beats | 48 (1.5) | 38(1.2) | ns |
| | | | |
| Sustained VT(>= 30 sees) | 14 (0.44) | 14(0.44) | ns |
| Monomorphic | 7 (0.22) | 4 (0.12) | ns |
| Polymorphic | 7 (0.22) | 10 (0.32) | ns |
| | | | |
| ----------------------------------------------------- | --------------- | ----------------- | ------------------- |
| | | | |
| Brady, Complete heart block, or Pause >=2.5 see | 1485 (45.4) | 1257 (38.5) | <0.001 |
| | | | |
| Bradycardia < 45 bpm for at least 4 beats | 1460 (44.6) | 1236 (37.8) | <0.001 |
| | | | |
| Pause > 3 sees | 136(4.3) | 97 (3.1) | 0.01 |
| Sinoatrial node block | 82 (2.6) | 64 (2.0) | ns |
| AV node block | 46 (1.4) | 31 (1.0) | ns |
| Other | 7 (0.2) | 2 (0.1) | ns |
| | | | |

Specifically, fewer patients had an episode of ventricular arrhythmia lasting >=8 beats (175 [5.5%] v. 277 [8/7%], p<0.001) (see Figure 20), supraventricular tachycardia (1413 [43.2%] v. 1752 [53.5%], p<0.001) or new-onset atrial fibrillation (55 [1.7%] v. 75 [2.3%], p=0.09). In addition, pauses >= 3 seconds (97 [3.1%] v. 136 [4.3%], p=0.01) and bradycardia <45 bpm were less frequent with ranolazine (as shown in the bottom half of the above table). Clinically reported sudden cardiac death was also numerically lower in the group allocated to ranolazine (56 v. 65, p-0.43).

### Conclusion

Ranolazine, an inhibitor of the late phase of the sodium current, appears to have anti-arrhythmic effects as assessed by continuous ECG monitoring in the first week after admission for ACS.

Of the 6560 patients in MERLIN - TIMI 36, the incidence of ventricular tachyarrhythmias detected on cECG monitoring after NST elevation myocardial infarction is shown in the below table.

| | Placebo n (%) | Δ (%) | Ranolazine n (%) | p value |
|---|---|---|---|---|
| | | | | |
| VT ≥3 beats (≥100 b/m) | 1993 (60.6) | 347 (-17) | 1646 (52.1) | <0.001 |
| VT ≥4 beats (≥100 b/m) | 941 (29.5) | 279 (-30) | 662 (20.9) | <0.001 |
| VT ≥8 beats (< 30 see) | 265 (8.3) | 99 (-37) | 166 (5.3) | <0.001 |
| Polymorphic VT >= 8 beats | 48 (1.5) | 10 (-21) | 38 (1.2) | ns |
| Sustained VT (>=30 sec) | 14 (0.44) | 0 | 14 (0.44) | ns |

The incidence of ventricular tachycardia in the MERLIN - TIMI 36 Trial is shown in the below table.

| | Incidence (%) | | |
|---|---|---|---|
| No. of beats | Placebo | Ranolazine | p value |
| | | | |
| ≥8 | 8.3% | 5.3% | <0.001 |
| ≥10 | 5.7% | 3.5% | <0.001 |
| ≥15 | 2.5% | 1.3% | <0.001 |
| ≥20 | 1.3% | 0.9% | <0.07 |

The incidence of ventricular tachycardia of >8 beats in subgroups of patients in the MERLIN - TIMI 36 trial is shown in the below two tables.
Subgroup: ejection fraction

| | Incidence (%) | | |
|---|---|---|---|
| Ejection fraction (%) | Placebo | Ranolazine | p value |
| | | | |
| ≥40 | 7.3% | 5.3% | <0.001 |
| <40 | 16.6 % | 8.8% | =0.001 |

Subgroup: baseline QT_{c}

| | Incidence (%) | | |
|---|---|---|---|
| Baseline QT_{c} (msec) | Placebo | Ranolazine | p value |
| | | | |
| ≥450 | 7.8% | 5.2% | <0.001 |
| > 450 | 10.5% | 5.6% | =0.013 |

### EXAMPLE 9

### The Effect of Ranolazine, a Novel Anti-anginal Agent with Electrophysiologic Properties, on the Incidence of Arrhythmias after Non-ST-segment Elevation Acute Coronary Syndrome:

### Results from the MERLIN-TIMI 36 Randomized Clinical Trial

### Background

Ranolazine reduces ischemia via inhibition of the late phase of the inward sodium current during cardiac repolarization, with a consequent reduction in intracellular sodium and calcium overload. Increased intracellular calcium leads to both mechanical and electrical myocyte excitability. Ranolazine reduces pro-arrrhythmic substrate and triggers such as early after-depolarizations in animal models.

### Methods

The MERLIN-TIMI 36 trial randomized 6560 patients hospitalized with a non-ST elevation acute coronary syndrome to ranolazine or placebo in addition to standard therapy. Continuous ECG (cECG or Holter) recording was performed for the first 7 days after randomization. Prespecified arrhythmia analyses were evaluated by a core laboratory blinded to treatment and outcomes.

### Results

Of the 6560 patients in MERLIN-TIMI 36, 6351 patients (97%) had a cECG recording valid for arrhythmia analysis. Treatment with ranolazine resulted in significantly lower rates of arrhythmias. Specifically, fewer patients had an episode of ventricular tachycardia lasting > 8 beats (166 [5.3%] v. 265 [8.3%], p<0.001), supraventricular tachycardia (1413 [44.7%] v. 1752 [55.0%], p<0.001) or new-onset atrial fibrillation (55[1.7%] v. 75 [2.4%], p=0.08). In addition, pauses > 3 seconds were also less frequent with ranolazine (97 [3.1%] v. 136 [4.3%], p=0.01).

### Conclusion

In over 6300 patients admitted with non-ST-elevation acute coronary syndrome, treatment with ranolazine resulted in significantly lower rates of ventricular tachycardia, supraventricular tachycardia, and significant ventricular pauses.

Further findings from the MERLIN-TIMI 36 study are presented herewith in Appendix A, which is incorporated herein by reference in its entirety.

### EXAMPLE 10

### Effect of Ranolazine on Hyperglycemia in the MERLIN-TIMI 36 Randomized Controlled Trial

### Background

A prospective evaluation of the effect of ranolazine on hyperglycemia as part of a randomized, double-blind, placebo-controlled trial in acute coronary syndromes (ACS).

### Methods

MERLIN-TIMI 36 randomized patients with non-ST elevation ACS to ranolazine or placebo to compare HbA1c (%) and the time to onset of worsening hyperglycemia (>1% increase in HbAlc). HbA1c data are reported as least-square means. Patients categorized as "diabetic" had been diagnosed as diabetic before or at the time of randomization. Patients categorized as "no diabetes" had not been diagnosed as diabetic before or at the time of randomization. Some patients characterized as "no diabetes" may have been diagnosed as "diabetic" during the trial; however, these patients are still listed in the "no diabetes" category in Figure 4B.

### Results

Among 4306 patients with serial measurements, ranolazine significantly reduced HbA1c at 4 months compared with placebo (5.9% vs. 6.2%, change from baseline -0.30 vs. -0.04 p=0.001). In patients with DM treated with ranolazine, HbA1c declined from 7.2 to 6.8 (Δ -0.64, p<0.001, see Figure 21A). As such, patients with DM were significantly more likely to achieve an HbA1c <7% at 4 months when treated with ranolazine versus placebo (59% vs. 49%, p<0.001). In addition, worsening of hyperglycemia by 1 year of follow-up was less likely in diabetic patients treated with ranolazine (14.2% vs. 20.6%; HR 0.63; 95% CI 0.51, 0.77, p<0-001). Notably, in patients without DM at randomization or baseline (fasting glucose <100 mg/dL and HbA1c <6%), the incidence of new fasting glucose >110 mg/dL or HbA1c ≥6% was also reduced by ranolazine (31.8% vs. 41.2%; HR 0.68; 95% CI 0.53, 0.88; p=0.003; see Figure 21B). Reported hypoglycemia in patients with DM was similar between treatment groups (3% vs 3%).

### Conclusion

Ranolazine significantly improved HbA1c in patients with DM and reduced the incidence of newly increased HbA1c in those without evidence of previous hyperglycemia.

### EXAMPLE 11

### Efficacy and Safety of Ranolazine in Women with Non-ST Elevation Acute Coronary Syndromes in MERLIN-TIMI 36

### Background

The pathobiologic basis of cardiovascular disease, and thus the response to medical therapy, can differ between women and men. In prior studies, sex-based treatment differences were observed with ranolazine, with a possibly diminished effect in women. Additionally, it has been proposed that women have similar or possibly more favorable outcomes then men after non-ST elevation ACS (NSTE ACS).

### Methods

In the MERLIN-TIMI 36 study the characteristics and clinical outcomes of women with NSTE ACS randomized to ranolazine or placebo were studied over a 1 year period.

### Results

Compared with men (N=4,269), women (N=2,291) were older and had higher rates of diabetes, hypertension, prior heart failure, and prior angina (P<0.001 for each). On presentation, women were more likely than men to have to have ST depression ≥0.1 mV (40.9 vs. 32.0%, P<0.001) and elevated BNP (47.0 vs. 40.2%, P<0.001), yet they were less likely to have evidence of epicardial CAD (no stenosis on angiogram: 19.4 vs. 8.6%, P<0.001) or elevated troponin (57.1 va. 68.9%, P<0.001). Despite these differences, women and men were at similar risk for the primary endpoint of CV death, MI, or recurrent ischemia (adj HR (women:men) 1.06, 95%CI 0.96 -1.18). There was no effect on major cardiovascular endpoints in the overall study analysis, but exploratory analyses provided evidence for an anti-anginal effect of ranolazine. Treatment with ranolazine was associated with a significant reduction in worsening angina in women (1-year incidence 3.9 vs. 6.8%, HR 0.61, 95%CI 0.42 -0.89, P=0.01), driven by a reduction in recurrent ischemia (see Figure 22). No difference in symptomatic documented arrhythmias was observed in women treated with ranolazine vs placebo (2.6 vs 2.6%, P=0.95).

### Conclusion

Women with a clinical syndrome consistent with ACS were less likely than men to have obstructive epicardial CAD but were at a similar risk of CV events, including recurrent ischemia.

### EXAMPLE 12

### Prognostic Implications of Low Level Elevation of Cardiac Troponin Using a New Ultra-sensitive Assay for Cardiac Troponin I: Results from the MERLIN-TIMI 36 Trial

### Background

Present recommendations define myocardial infarction (MI) using a cut-point for troponin at the 99^{th} percentile of a control population. The availability of new, more sensitive assays has enabled detection of increasingly lower concentrations of troponin, raising questions as to the clinical relevance of such very low-level increases.

### Methods

Serum cardiac troponin I (cTnI) levels were measured using a recently available new generation assay (TnI-Ultra, Siemens Medical Solutions) at baseline in 4,513 patients with suspected non-ST elevation acute coronary syndromes (NSTE-ACS) randomized to ranolazine or placebo in the MERLIN-TIMI 36 trial. Patients were stratified using decision limits at the 99^{th} percentile (0.04 µg/L), and a WHO cut-point relative to CK-MB (1.5 mg/ml).

### Results

Patients with a baseline concentration of cTnI >=0.04 µg/L (N= 2924) were at a higher risk of death/MI at 30 days than patients with a negative cTnI result (6.1 vs 2.0%, p<0.001). After adjusting for all other elements of the TIMI Risk Score for NSTE-ACS, baseline cTnI >=0.04 µg/L was associated with a 3.0 fold (95% CI 2.0-4.4, p<0.001) higher risk of death/MI at 30 days. Moreover, those patients with very low level increases detectable with the newer generation assay, cTnI (0.04-<0.1 µg/L), were at significantly higher risk of death/MI at 30 days than those without elevation (5.0 vs 2.0%, p=0.001) and only modestly lower than those with cTnI >=0.1 µg/L (6.2%). This significant relationship persisted at 1 year (see Figure 23). There was no evidence for heterogeneity in the effect of ranolazine compared with placebo between patients with and without elevation of cTnI.

### Conclusions

Low-level increases in cTnI using a new highly sensitive assay identify patients at significantly increased risk of death or MI. These findings support contemporary evolution of AHA/ACC recommendations defining MI, and the incremental value of new more sensitive assays in identifying high risk patients with suspected NSTE-ACS.

### EXAMPLE 13

### Baseline Clinical Risk and Recurrent Ischemia as Detected on Continuous ECG (CECG) Monitoring in Patients with non-ST Elevation Acute Coronary Syndrome in the MERLIN-TIMI 36 Trial

### Background

This Study evaluated the association between TIMI Risk Score (TRS) for NSTEACS (US/NSTEMI) and subsequent ischemia detected on CECG monitoring.

### Methods

The MERLIN-TIMI 36 study randomized 6560 patients with NSTEACS to the anti-ischemic agent ranolazine or placebo. Median clinical follow-up was approximately 12 months. At randomization, 3-lead CECG monitoring was initiated for a median of 6.9 days. Recurrent ischemia on CECG was defined as ST dep 1 mm lasting 1 minute. The TRS was calculated as the sum of 7 presenting characteristics (age ≥ 65 yes, ≥ 3 cardiac risk factors, documented CAD, recent severe angina, ST dev ≥ 5 mm, elevated cardiac markers, prior ASA use) and categorized as low (0-2), moderate (3-4), or high (>4).

### Results

A total of 30.2% of the patients were low risk (TRS 0-2), a52.5% were moderate risk TRS (3-4) and 17.3% were high risk (TRS 5-7). Ischemia was detected on CECG in 1239/6355 (19.5%) patients with 633 (10.0%) experiencing >2 episodes. Patients with higher TRS were more likely to experience any ischemic episode (13.5% in low TRS v 19.1% in moderate TRS v 31.1 % in high TRS, p<0.001) and >2 episodes (5.5% v 10.1% v 17.3%, p<0.001). Among patients who experienced ischemia, those with higher TRS had a longer total duration of ischemia (66.5 v 102.3 v 115.5 min. p<0.001). Overall and within each TRS risk category, ischemia detected on CECG was associated with worse CV outcome (see Figure 24).

### Conclusions

Recurrent ischemia as detected on CECG was more frequent among patients with higher clinical risk as determined by TRS. Even among patients with a similar baseline risk by TRS, subsequent recurrent ischemia on CECG was associated with worse long-term cardiovascular outcomes.

### EXAMPLE 14

### Ranolazine Reduces Ischemia as Detected on Continuous ECG (Holter) in Patients with non-ST Elevation ACS in the MERLIN-TIMI 36 Trial

### Background

Recurrent ischemia after admission for non-STE ACS is common and associated with poor outcomes. In patients with chronic CAD, ranolazine, a novel anti-anginal agent, improves symptoms and delays the time until ST depression during stress tests. Continuous ECG (CECG) monitoring is a sensitive marker of recurrent ischemia.

### Methods

In the MERLIN-TIMI 36 trial 6560 patients admitted with NSTEACS were randomized to ranolazine or placebo. At randomization, 6355 patients (99%) had 3-lead CECG monitoring initiated (median duration 6.9 days). The primary CECG endpoint was the rate of ST dep ≥1 mm from baseline lasting ≥ 1 minute with heart rate at onset <100 bpm. Exploratory analyses using ST dep ≥0.5 mm were also performed.

### Results

Ranolazine did not reduce the rate of the primary CECG endpoint compared to placebo (19.2 v 20.5%, RR 0.03, p=0.18) through the entire 7 day recording though it did reduce events >72 hours after randomization (11.6 v 13.6%, RR 0.86, p=0.02). When using the lower ischemic threshold of 0.5 mm, however, ranolazine did reduce events (22.9 v 25.1, RR 0.91, p=0.039). This effect was consistent regardless of HR at the onset of ischemia, but the reduction appeared more prominent with elevated HR (see Figure 25).

### Conclusion

Ranolazine, a novel anti-anginal agent, appeared to reduce the rate of ischemia as detected by CECG in patients with NSTEACS using a more sensitive ECG cutpoint for ischemia, in particular several days after randomization and episodes that started with an increased HR. This suggests that the greatest anti-anginal effect of ranolazine may be to diminish "demand-related" ischemia.

## Claims

1. Ranolazine for use in a method for treating bradycardia or bradyarrythmia in a patient.

2. The ranolazine of claim 1, which is in the form of a pharmaceutically acceptable salt or in the form of the free base.

3. The ranolazine of claim 2, wherein the pharmaceutically acceptable salt is the dihydrochloride salt.

4. The ranolazine of any one of claims 1 to 3, which is administered as an IV ranolazine solution or as an oral formulation.

5. The ranolazine of claim 4, wherein administration of the IV solution to the patient is continued until the patient has been stabilized.

6. The ranolazine of claim 1, wherein the method comprises:
a) initiating administration of an IV solution of ranolazine to the patient wherein said IV solution comprises a concentration of ranolazine of from about 1.5 to about 3 mg per milliliter,
b) titrating the IV administration of the IV ranolazine solution to the patient comprising:
i) a sufficient amount of the IV solution to provide for about 200 mg of ranolazine delivered to the patient over about a 1 hour period;
ii) followed by either: a sufficient amount of the IV solution to provide for about 80 mg of ranolazine per hour; or if said patient is suffering from renal insufficiency, a sufficient amount of the IV solution to provide for about 40 mg of ranolazine per hour; and
c) maintaining the titration of b) above until the patient has been stabilized.

7. The ranolazine of Claim 6, wherein the IV ranolazine solution comprises from about 1.8 to about 2.2 mg ranolazine per milliliter of solution.

8. The ranolazine of Claim 5 or 6, wherein stabilization occurs within from about 12 to about 96 hours after initiation of the IV administration.

9. The ranolazine of Claim 6, wherein the administration of the intravenous formulation of ranolazine is initiated such that a target peak ranolazine plasma concentration of about 2500 ng base/mL is achieved.

10. The ranolazine of Claim 6, wherein after the patient is stabilized, the patient is then transitioned from the intravenous to an oral dose by administering an oral sustained release formulation of ranolazine.

11. The ranolazine of Claim 10, wherein the oral dose of ranolazine is administered about 1 hour prior to the termination of the intravenous infusion of ranolazine.

12. The ranolazine of Claim 10, wherein at the time of transition from intravenous to oral dose, for the intravenous dose of ranolazine of about 80 mg/hr, the oral dose administered is 1000 mg twice daily (2 x 500 mg).

13. The ranolazine of Claim 10, wherein at the time of transition from intravenous to oral dose, for the intravenous dose of ranolazine of about 60 mg/hr, the oral dose administered is 750 mg twice daily (2 x 375 mg).

14. The ranolazine of Claim 10, wherein at the time of transition from intravenous to oral dose, for the intravenous dose of ranolazine of about 40 mg/hr, the oral dose administered is 500 mg twice daily (1 x 500 mg).

15. The ranolazine of Claim 10, wherein at the time of transition from intravenous to oral dose, for the intravenous dose of ranolazine of about 30 mg/hr, the oral dose administered is 375 mg twice daily (1 x 375 mg).
